# EUROPEAN PATENT SPECIFICATION

(11) **EP 2 897 634 B1**
(45) Date of publication and mention of the grant of the patent: **21.03.2018**
(21) Application number: 13837157.0
(22) Date of filing: 12.09.2013
(51) Int. Cl.: C07K 14/755, C12N 9/64, C12N 15/12, A61K 38/37, A61P 7/04, C12N 15/57

(54) **DOUBLE MUTANT COAGULATION FACTOR VIII AND METHODS THEREOF**
DOPPELT MUTIERTER BLUTGERINNUNGSFAKTOR VIII UND VERFAHREN DAFÜR
FACTEUR VIII DE COAGULATION À DOUBLE MUTATION ET PROCÉDÉS ASSOCIÉS

(30) Priority: 12.09.2012 IN 3778CH2012
(43) Date of publication of application: 29.07.2015
(73) Proprietor: Centre for Bioseparation Technology-VIT, Tamil Nadu 632014 (IN); Christian Medical College, 632 004 Tamil Nadu (IN)
(72) Inventor: ARUNACHALAM, Vijayalakshmi Mookambeswaran, Vellore 632014 (IN); JOSEPH, Bilgimol Chuvappumkal, Vellore 632014 (IN); KUTTY, Satheeshkumar Padikkara, Vellore 632014 (IN); NAIR, Sukesh Chandran, Vellore 632004 (IN); SRIVASTAVA, Alok, Vellore 632004 (IN)
(74) Representative: V.O.
(86) International application number: PCT/IB2013/058490
(87) International publication number: WO 2014/041500

(56) References cited:
- WO-A1-97/40145
- WO-A1-2010/115866
- WO-A2-98/00541
- US-A1- 2012 065 136
- US-A1- 2012 190 623
- WAKABAYASHI H ET AL: "Combining mutations of charged residues at the A2 domain interface enhances factor VIII stability over single point mutations.", JOURNAL OF THROMBOSIS AND HAEMOSTASIS : JTH MAR 2009, vol. 7, no. 3, March 2009 (2009-03), pages 438-444, XP002755736, ISSN: 1538-7836
- MARQUETTE KIMBERLY A ET AL: "A 110-amino Acid Region within the A1-domain of Coagulation Factor VIII Inhibits Secretion from Mammalian Cells", JOURNAL OF BIOLOGICAL CHEMISTRY, vol. 270, no. 17, 1995, pages 10297-10303, XP2157395, ISSN: 0021-9258
- SWAROOP M ET AL: "Mutagenesis of a potential immunoglobulin-binding site enhance", JOURNAL OF BIOLOGICAL CHEMISTRY, AMERICAN SOCIETY FOR BIOCHEMISTRY AND MOLECULAR BIOLOGY, US, vol. 272, no. 39, 1 January 1997 (1997-01-01), pages 24121-24124, XP002988831, ISSN: 0021-9258, DOI: 10.1074/JBC.272.39.24121

## Description

### TECHNICAL FIELD

The present disclosure is in the field of haemophilia therapeutics, particularly recombinant Factor VIII protein products. The present disclosure relates specifically to an expression cassette comprising a nucleotide sequence coding for double mutant B-domain deleted Factor VIII gene having mutations at Phe309Ser and Asp519Val respectively. The disclosure also provides vectors and host cells transformed by the said expression cassette and corresponding methods thereof. The disclosure also relates to the production of double mutant B-domain deleted Factor VIII protein having mutations at Phe309Ser and Asp519Val respectively. The said protein shows enhanced activity and stability and therefore is used in the management of haemophilia.

### BACKGROUND AND PRIOR ART OF THE DISCLOSURE

Hemophilia A is a congenital or acquired disorder of coagulation that usually involves quantitative or functional disorder of a single coagulation protein. The available treatment for hemophilia A is the administration of plasma derived factor VIII or the infusion of recombinant factor VIII.

Factor VIII (FVIII) has 26 exons, encoding a polypeptide chain of 2351 amino acids (19 amino acids signal peptide and 2332 amino acids mature protein). It is a large multimeric protein with heavy (A1-A2-B domains) and light chains (A3-C1-C2 domains). The domain structure of factor VIII is arranged in order as (NH2)-A1-a1-A2-a2-B-a3-A3-C1-C2-(COOH) (a1, a2 and a3 indicate the spacers containing clusters of acidic amino acids). The molecular weight of heavy chain spans between 90 and 210 kDa due to the limited processing of several proteolytic sites in B domain. The molecular weight of light chain is 80 kDa as this is not processed (Kaufman et al, 1988, Bovenschen et al, 2005). Heavy and light chains remain associated with each other with the non-covalent linkage of metal ion. (Vehar et al, 1984, Fass et al, 1982, Fay et al, 1986, Fay et al, 1992).

The expression level of factor VIII in heterologous systems is lower than that of other similar proteins expressed (Kaufman, et al. 1997). The reasons behind the limited expression of factor VIII in heterologous systems are: inefficient expression of factor VIII mRNA, ineffective transport of translated product from endoplasmic reticulum to the Golgi apparatus, interaction of misfolded proteins with chaperons in the ER etc.

Marquette et al identified a 110 amino acid region within the A1 domain of factor VIII which inhibited factor VIII secretion from the ER (Marquette, et al. 1995). Further to this, the structural analysis revealed the presence of a hydrophobic β-sheet within this region (Swaroop, et al. 1997). Measures were taken to reduce the interaction of factor VIII with immunoglobulin-binding protein (BiP) by mutating the potential amino acids to hydrophilic amino acids (Swaroop, et al. 1997).

In the prior art, different mutations (single, double and triple) are reported pertaining to improve a single feature/trait. The mutations generated are aimed to improve the stability of the recombinant Factor VIII molecule by single [single mutations at the sites 519, 665 (A2 domain), and 1984 (A3 domain)], double [eleven double mutations in different combinations at the sites of 519, 665, and 1984] and triple [three triple mutations at the sites of 519, 665, and 1984 sites]. Further, the prior art also deals with the mutations aiming to improve the secretion of recombinant Factor VIII and concerned only about the amino acids in the A1 domain which interact with the proteins in the ER secretory pathway. The mutations generated are single [nine single mutations at the positions 293, 294, 300, 309, 310, 306, and 299 (present in the 110 amino acid stretch in A1 domain which interacts with chaperons)], double [five double mutations with different combinations of double mutations at the positions 293, 294, 300, 309, 310, 306, and 299], and triple mutations.

US20120190623 discloses a truncated B-domain FVIII carrying the mutation F309S.

US2012065136 discloses a mutant FVIII with multiple mutations e.g. D519V.

The point 'synergism' is applicable only if the mutations considered are pertaining to a single character. The activity of the recombinant Factor VIII is ultimately decided by the amount of protein successfully secreted with significant stability. The prior art so far has not reported any studies which tried to combine the two features- secretion and stability. Thus, the present disclosure aims at overcoming the drawbacks of the prior art by disclosing double mutant showing improved activity due to the combined features of increased stability and secretion.

### STATEMENT OF THE DISCLOSURE

Accordingly, the present disclosure relates to a nucleotide sequence set forth as SEQ ID No. 1 or a nucleotide sequence comprising sequence set forth as SEQ ID No.1; an amino acid sequence set forth as SEQ ID No. 2 or an amino acid sequence comprising sequence set forth as SEQ ID No.2; an expression cassette comprising nucleotide sequence set forth as SEQ ID No. 1 or a nucleotide sequence comprising sequence set forth as SEQ ID No.1; a vector having an expression cassette of the present disclosure; a host cell comprising the vector of the present disclosure; a method of obtaining a nucleotide sequence set forth as SEQ ID No. 1 or its corresponding amino acid sequence, said method comprising act of mutating nucleotide sequence set forth as SEQ ID NO. 3 at positions 925-927 and 1555-1557 or mutating a corresponding amino acid sequence set forth as SEQ ID NO. 4 at positions 309 and 519; a method of obtaining a transformed host cell comprising an expression cassette of the present disclosure, said method comprising acts of inserting the expression cassette of the present disclosure into a vector; and transforming the host cell with said vector to obtain the transformed host cell; a nucleotide sequence as set forth in SEQ ID No.13; an amino acid sequence as set forth in SEQ ID NO. 14; a protein having amino acid sequence set forth as SEQ ID No. 14; a method of producing a protein having amino acid sequence set forth as SEQ ID No. 14, said method comprising acts of mutating 925-927 and 1555-1557 positions of nucleotide sequence set forth as SEQ ID No. 3 to obtain a nucleotide sequence set forth as SEQ ID No. 1; transforming a host cell comprising vector having an expression cassette comprising the nucleotide sequence set forth as SEQ ID No. 1; and culturing the transformed host cell in culture medium for producing the said protein; a composition comprising protein having amino acid sequence set forth as SEQ ID No. 14 optionally along with excipients; a kit comprising mature recombinant double mutant factor VIII protein having amino acid sequence set forth as SEQ ID No. 14; a method of enhancing plasma deficient in factor VIII, said method comprising act of addition of mature recombinant double mutant factor VIII protein having amino acid sequence set forth as SEQ ID No. 14 to the plasma deficient in factor VIII; a method of activating factor X, said method comprising act of incubating factor X or a sample comprising factor X with mature recombinant double mutant factor VIII protein having amino acid sequence set forth as SEQ ID No. 14 to activate the factor X; and a method of managing coagulation disorders, said method comprising act of administering to a subject in need thereof, a composition comprising a protein having amino acid sequence set forth as SEQ ID No. 14, optionally along with pharmaceutically acceptable carrier or excipient or a combination thereof.

### BRIEF DESCRIPTION OF ACCOMPANYING FIGURES

In order that the invention may be readily understood and put into practical effect, reference will now be made to exemplary embodiments as illustrated with reference to the accompanying figures. The figures together with a detailed description below, are incorporated in and form part of the specification, and serve to further illustrate the various embodiments, principles and advantages, in accordance with the present disclosure where:
**Figure 1** depicts the mutations carried out at the various sites of the BDD recombinant Factor VIII gene.
**Figure 2** depicts the western blot confirmation of wild-type Factor VIII (represented as BDD in the figure) and mutated recombinant Factor VIII (DM1 and DM2). The cell culture media collected after 48 and 72 hours is used in the experiment. First four lanes (1-4) indicate samples collected for two clones of double mutant and lane 5 and 6 represent wild-type BDD-FVIII protein.
   Legend for Figure - 2:
   Lane 1 and 2: protein expressed at 48 hours and 72 hours (DM-1)
   Lane 3 and 4: protein expressed at 48 hours and 72 hours (DM-2)
   Lane 5 and 6: protein expressed at 48 hours and 72 hours (wild-type BDD-FVIII without mutation). Arrows indicate the full length BDD-F VIII (∼180KDa) and Light chain of F VIII (∼70KDa). DM=Double mutant recombinant Factor VIII and BDD= B Domain Deleted wild-type Factor VIII without mutation.

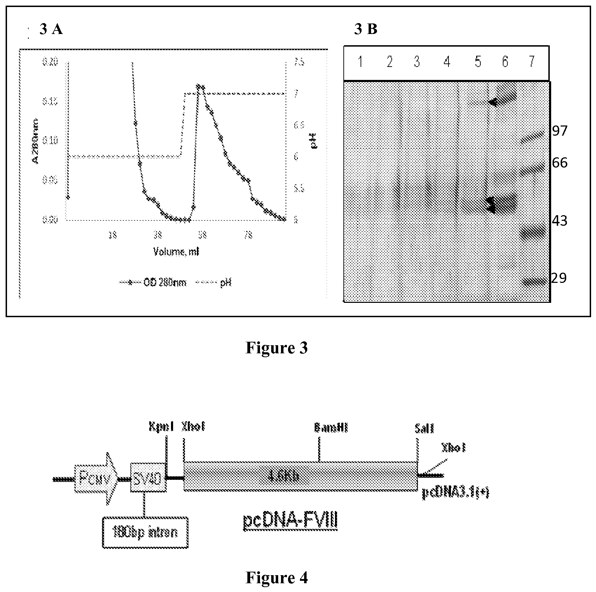

**Figure 3** depicts purification of recombinant ΔBDD-FVIII using histidine ligand affinity chromatography. The chromatogram (3A) shows a single peak with the elution buffer. Purified fractions are then subjected to SDS-PAGE analysis (3B). Lane 1=load, lane 2=flow through, lane 3=wash, lane 4=elution without concentrating, lane 5=elution after concentrating, lane 6=commercial Factor VIII, lane 7=medium range protein molecular weight markers. The arrows indicate the full length and individual chains of Factor VIII.
**Figure 4** depicts the pcDNA 3.1 vector construct.

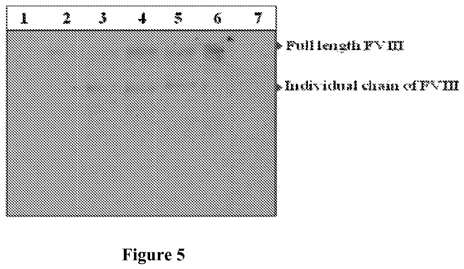

**Figure 5** depicts the Western blot analysis of purified fractions. Lane 1 - CHO, lane 2 - BDD-FVIII, Lane 3 & Lane 4 - double mutant, lane 5 - commercial factor VIII, Lane 6 - D519V.

### DETAILED DESCRIPTION OF THE DISCLOSURE

The present disclosure relates to a nucleotide sequence set forth as SEQ ID NO. 1 or a nucleotide sequence comprising sequence set forth as SEQ ID NO.1.

In an embodiment of the present disclosure, the nucleotide sequence of SEQ ID NO. 1 corresponds to B-domain deleted Factor VIII gene and comprises mutations at 925-927 and 1555-1557 positions of said nucleotide sequence; wherein the mutation in the nucleotide sequence at the position 925-927 corresponds to the replacement of phenylalanine with serine at 309 position in the corresponding amino acid sequence and the mutation in the nucleotide sequence at the position 1555-1557 corresponds to the replacement of aspartic acid with valine at 519 position in the corresponding amino acid sequence.

In an embodiment of the present disclosure, the mutations are point mutations at 925, 926 and 1556 position of said nucleotide sequence.

The present disclosure also relates to an amino acid sequence set forth as SEQ ID NO. 2 or an amino acid sequence comprising sequence set forth as SEQ ID NO.2.

In an embodiment of the present disclosure, the amino acid is corresponding to the nucleotide sequence of SEQ ID NO. 1.

In an embodiment of the present disclosure, the amino acid sequence corresponds to the B-domain deleted Factor VIII protein and comprises mutations at 309 and 519 positions.

The present disclosure also relates to an expression cassette comprising nucleotide sequence set forth as SEQ ID NO. 1 or a nucleotide sequence comprising sequence set forth as SEQ ID NO.1.

In an embodiment of the present disclosure, the nucleotide sequence corresponds to B-domain deleted Factor VIII gene and comprises mutations at 925-927 and 1555-1557 positions of said nucleotide sequence; wherein the mutation in the nucleotide sequence at the position 925-927 corresponds to the replacement of phenylalanine with serine at 309 position in the corresponding amino acid sequence and the mutation in the nucleotide sequence at the position 1555-1557 corresponds to the replacement of aspartic acid with valine at 519 position in the corresponding amino acid sequence.

The present disclosure also relates to a vector having an expression cassette of the present disclosure.

In an embodiment of the present disclosure, the vector comprising SEQ ID NO. 1 is inserted into an *E.coli* deposited under accession number MTCC 5855.

The present disclosure also relates to a host cell comprising the vector of the present disclosure.

The present disclosure also relates to a method of obtaining a nucleotide sequence set forth as SEQ ID NO. 1 or its corresponding amino acid sequence, said method comprising act of mutating nucleotide sequence set forth as SEQ ID NO. 3 at positions 925-927 and 1555-1557 or mutating a corresponding amino acid sequence set forth as SEQ ID NO. 4 at positions 309 and 519.

In an embodiment of the present disclosure, the nucleotide sequence set forth as SEQ ID NO. 3 corresponds to wild-type B-domain deleted Factor VIII gene and wherein the corresponding amino acid sequence set forth as SEQ ID NO. 4 corresponds to the corresponding wild type B-domain deleted Factor VIII protein.

In an embodiment of the present disclosure, the mutation in the nucleotide sequence at position 925-927 corresponds to the replacement of phenylalanine with serine at 309 position in the corresponding amino acid sequence; and the mutation in the nucleotide sequence at position 1555-1557 corresponds to the replacement of aspartic acid with valine at 519 position in the corresponding amino acid sequence

The present disclosure relates to a method of obtaining a transformed host cell comprising an expression cassette or a vector comprising nucleotide sequence set forth as SEQ ID NO. 1 or a nucleotide sequence comprising sequence set forth as SEQ ID NO.1, said method comprises inserting the expression cassette into a vector; and transforming the host cell with said vector to obtain the transformed host cell.

In an embodiment of the present disclosure, the host cell is CHO cell.

The present disclosure also relates to a nucleotide sequence as set forth in SEQ ID NO.13.

In an embodiment of the present disclosure, the sequence corresponds to B-domain deleted Factor VIII gene and comprises mutations at 925-927 and 1555-1557 positions of said nucleotide sequence; wherein the mutation in the nucleotide sequence at the position 925-927 corresponds to the replacement of phenylalanine with serine at 309 position in the corresponding amino acid sequence and the mutation in the nucleotide sequence at the position 1555-1557 corresponds to the replacement of aspartic acid with valine at 519 position in the corresponding amino acid sequence.

The present disclosure also relates to an amino acid sequence as set forth in SEQ ID NO. 14.

In an embodiment of the present disclosure, the amino acid sequence corresponds to the mature BDD FVIII double mutant peptide.

In an embodiment of the present disclosure, the amino acid sequence corresponds to the mature BDD Factor VIII mutant peptide and comprises mutations F309S and D519V.

The present disclosure also relates to a protein having amino acid sequence set forth as SEQ ID No. 14.

In an embodiment of the present disclosure, the amino acid sequence set forth as SEQ ID No. 14 correspond to mature BDD Factor VIII protein and comprises mutation F309S and D519V.

The present disclosure also relates to a method of producing a protein having amino acid sequence set forth as SEQ ID No. 14, said method comprises mutating 925-927 and 1555-1557 positions of nucleotide sequence set forth as SEQ ID No. 3 to obtain a nucleotide sequence set forth as SEQ ID No. 1; transforming a host cell comprising vector having an expression cassette comprising the nucleotide sequence set forth as SEQ ID No. 1; and culturing the transformed host cell in culture medium for producing said protein.

In an embodiment of the present disclosure, the amino acid sequence set forth as SEQ ID No. 14 corresponds to mature B-domain deleted Factor VIII protein and comprises mutations F309S and D519V.

The present disclosure also relates to a composition comprising protein having amino acid sequence set forth as SEQ ID No. 14 optionally along with excipients.

In an embodiment of the present disclosure, the composition further comprise divalent metal ion(s).

In an embodiment of the present disclosure, the divalent metal ion(s) is, but not limiting to, preferably Ca²⁺ ions.

In an embodiment of the present disclosure, the composition further comprise coagulation factors.

In an embodiment of the present disclosure, the said amino acid sequence comprise mutations F309S and D519V.

The present invention also relates to a kit comprising mature recombinant double mutant factor VIII protein having amino acid sequence set forth as SEQ ID No. 14;
The present invention also relates to a method of enhancing plasma deficient in factor VIII, said method comprising act of addition of mature recombinant double mutant factor VIII protein having amino acid sequence set forth as SEQ ID No. 14 to the plasma deficient in factor VIII;
The present invention also relates to a in vitro method of activating factor X, said method comprises incubating factor X or a sample comprising factor X with mature recombinant double mutant factor VIII protein having amino acid sequence set forth as SEQ ID No. 14 to activate the factor X;
The present disclosure also relates to a method of managing coagulation disorders, said method comprising act of administering to a subject in need thereof, a composition comprising a protein having amino acid sequence set forth as SEQ ID No. 14, optionally along with pharmaceutically acceptable carrier or excipient or a combination thereof.

In an embodiment of the present disclosure, the coagulation disorder is, but not limited to heamophilia.

The present disclosure relates to double mutant recombinant Factor VIII.

In a preferred embodiment, the mutations in recombinant Factor VIII are at two amino acid positions Phe309Ser (A1 domain) and Asp519Val (A2 domain) respectively.

As used herein, the following sequences are set forth and followed in the present disclosure-SEQ ID NO. 1 (Total Length- 4377 nucleotides): Nucleotide Sequence coding for the double mutant recombinant BDD-Factor VIII having mutations at 925-927 and 1555-1557 positions respectively. The said nucleotide sequence contains 1-57 short signal peptide coding sequence. The mutations at SEQ ID NO. 1 are at position 925-927 and at position 1555-1557, wherein this position is to be calculated taking into account the short signal peptide coding sequence of 57 nucleotides at the beginning of SEQ ID NO.1.

SEQ ID NO. 2 (Total Length- 1458 amino acids): Amino acid sequence of the double mutant recombinant BDD-Factor VIII having mutations at Phe309Ser (A1 domain) and Asp519Val (A2 domain) respectively. The said amino acid sequence contains 1-19 short signal peptide sequence. The mutations at SEQ ID NO. 2 are at position 309 and at position 519, wherein this position is to be calculated taking into account the short signal peptide coding sequence of 19 amino acid residues at the beginning of SEQ ID NO. 2.

SEQ ID NO. 3 (Total Length- 4377 nucleotides): Nucleotide Sequence coding for the wild type BDD-recombinant Factor VIII. The said nucleotide sequence contains 1-57 short signal peptide coding sequence.

SEQ ID NO. 4 (Total Length- 1458 amino acids): Amino acid sequence of wild type BDD-recombinant Factor VIII. The said amino acid sequence contains 1-19 short signal peptide sequence.

SEQ ID NO. 5: Forward primer for F309S mutation.

SEQ ID NO. 6: Reverse primer for F309S mutation.

SEQ ID NO. 7: Forward primer for D519V mutation.

SEQ ID NO. 8: Reverse primer for D519V mutation.

SEQ ID NO. 9: (Total Length- 4377 nucleotides): Nucleotide Sequence of the single mutant recombinant BDD-Factor VIII having mutation at Phe309Ser (A1 domain). The said nucleotide sequence contains 1-57 short signal peptide coding sequence.

SEQ ID NO. 10: (Total Length- 1458 amino acids): Amino acid sequence of the single mutant recombinant BDD-Factor VIII having mutation at Phe309Ser (A1 domain). The said amino acid sequence contains 1-19 short signal peptide sequence.

SEQ ID NO. 11: (Total Length- 4377 nucleotides): Nucleotide Sequence of the single mutant recombinant BDD-Factor VIII having mutation at Asp519Val (A2 domain). The said nucleotide sequence contains 1-57 short signal peptide coding sequence.

SEQ ID NO. 12: (Total Length- 1458 amino acids): Amino acid sequence of the single mutant recombinant BDD-Factor VIII having mutation at Asp519Val (A2 domain). The said amino acid sequence contains 1-19 short signal peptide sequence.

SEQ ID NO. 13: (Total length 4320): Nucleotide sequence coding for the mature double mutant recombinant BDD-Factor VIII peptide having mutations at 925-927 and 1555-1557 positions respectively.

SEQ ID NO. 14: (Total length 1439): Amino acid sequence coding for the mature peptide for the double mutant recombinant BDD-factor VIII having mutation F309S and D519V.

In an embodiment of the present invention, the nucleotide sequence as set forth in SEQ ID No. 13 codes for the mature double mutant recombinant BDD-Factor VIII having mutations at positions 925-927 and 1555-1557. Further, the amino acid sequence as set forth in SEQ ID NO. 14 is the mature double mutant recombinant BDDFactor VIII having mutations F309S and D519V.

In an embodiment of the present disclosure, the nucleotide sequence as set forth in SEQ ID No. 1 codes for the double mutant recombinant BDD-Factor VIII containing mutations at positions 925-927 and 1555-1557 which also includes the sequence for the signal peptide.

Further, the amino acid sequence as set forth in SEQ ID NO. 2 is the double mutant recombinant BDD Factor VIII having mutations F309S and D519V including the amino acid sequence of the signal peptide.

As used herein, "management" or "managing" refers to preventing a disease or disorder from occurring in a subject, decreasing the risk of death due to a disease or disorder, delaying the onset of a disease or disorder, inhibiting the progression of a disease or disorder, partial or complete cure of a disease or disorder and/or adverse affect attributable to the said disease or disorder, obtaining a desired pharmacologic and/or physiologic effect (the effect may be prophylactic in terms of completely or partially preventing a disorder or disease or condition, or a symptom thereof and/or may be therapeutic in terms of a partial or complete cure for a disease or disorder and/or adverse affect attributable to the disease or disorder), relieving a disease or disorder (i.e. causing regression of the disease or disorder).

In an embodiment of the present disclosure, the double mutant recombinant Factor VIII showcases significantly improved secretion, increased circulating half life and stability and reduced immunogenicity.

In another embodiment of the present disclosure, the double mutant recombinant Factor VIII gene is expressed in mammalian heterologous expression systems not limited to CHO, BHK, sf9, HEK 293 F and Hep3B cell lines. In a preferred embodiment, the double mutant recombinant Factor VIII gene is expressed in CHO cell line.

In another embodiment of the present disclosure, the double mutant recombinant Factor VIII gene is expressed in pichia expression system or plant based expression system.

In an embodiment of the present disclosure, the double mutant recombinant Factor VIII gene is SEQ ID NO.1.

In an embodiment of the present disclosure, the double mutant recombinant Factor VIII gene is SEQ ID NO.13.

In an embodiment of the present invention, the double mutant recombinant Factor VIII gene set forth as SEQ ID NO.13 is inserted into a vector having sequence coding for signal peptide.

In an embodiment of the present invention, the double mutant recombinant Factor VIII of the present disclosure results in higher secretion, stability and activity. I. The double mutant F309S + D519V show an increased activity when compared to the wild type, single mutant F309S and D519V. The increased activity of the double mutant is explained by the synergistic effect of this combination (F309S + D519V) over the individual mutations F309S and D519V, wherein the functional roles of amino acids at these positions improve the secretion, stability and activity.

In an embodiment of the present disclosure, addition of Calcium ion in the formulation/composition comprising double mutant recombinant factor VIII increases the unit activity of double mutant recombinant Factor VIII (ΔBDD-FVIII). The increased activity observed with Ca²⁺ is due to the effect of this specific combination of F309S and D519V mutations together in a single construct. Serine at 309 position is important in metal ion interaction over phenylalanine when the next position is occupied by Cysteine (Cys310). A mutation at 519 changes the conformational positioning of A1 and A3 and makes more amenable to Factor IXa binding through metal ion interaction. So putting this together, the double mutant of the present disclosure shows more stability compared to wild type BDD-FVIII. Further, reconstitution buffer combinations with calcium increases the activity of this double mutant.

Addition of divalent metal ions to the recombinant preparations used for therapeutic purposes is not a common practice. The structural elucidation of Factor VIII molecule, its interaction between different domains reveal that metal ions play a vital role in maintaining its functional integrity. It is shown that when heavy chain and light chain of Factor VIII reconstitutes in the presence of divalent metal ions they regain coagulation activity. Nonetheless, there are no reports so far which show that the recombinant preparation of Factor VIII increases its activity by the addition of divalent cations.

In an embodiment of the present disclosure, the present disclosure utilizes the expected non-linked heavy chain and light chain in the preparations (as they are non-functional when separated) by adding 5mM CaCl₂ to facilitate the inter chain linking of heavy and light chain to make them functionally active. The mutation in A2 domain (D519V) significantly improves the inter domain interaction through calcium.

Specific mutations at crucial sites of any protein can alter its biological function. With regard to the therapeutic protein produced using heterologous system, the particular technique of the present study is used extensively to generate mutant proteins. Even though single mutations have been produced earlier, the double mutant of the present disclosure is significantly better in terms of its activity and stability. Thus, the approach of the present disclosure is based mainly on the practical aspects, concerning with the stability and secretion of Factor VIII molecule produced from CHO.

The double mutant of the present invention is referred throughout the specification interchangeably as double mutant B-domain deleted Factor VIII, double mutant (Phe309Ser + Asp519Va1), double mutant (F309S + D519V), double mutant BDD-FVIII, double mutant BDD-FVIII (Phe309Ser + Asp519Val), double mutant BDD-FVIII (F309S + D519V), double mutant recombinant factor VIII, recombinant BDD-FVIII- double mutant, rBDD-FVIII-DMand ΔBDD-FVIII.The expressed double mutant mature protein of the present invention contains the sequence as denoted by SEQ ID NO. 14, encoded by the nucleotide sequence of SEQ ID NO. 1. The double mutant protein of the present invention containing the sequence as denoted by SEQ ID NO. 2 corresponds to the nucleotide sequence of SEQ ID NO. 1.

The present invention discloses the double mutant BDD-FVIII (Phe309Ser + Asp519Val) which has been deposited with an International Depository Authority as per the requirement under Budapest treaty.

The cDNA construct in Escherichia coli- pcDNA for the recombinant BDD-FVIII- double mutant (rBDD-FVIII-DM) has been deposited with the International Depository, "The Microbial Type Culture Collection and Gene Bank (MTCC)" and has been accorded the accession number as **MTCC 5855.**

Additional embodiments and features of the present disclosure will be apparent to one of ordinary skill in art based upon description provided herein. However, the examples and the figures should not be construed to limit the scope of the present disclosure.

### Example 1

### Materials Used

*E. coli* strain DH5α is used for cloning and maintaining the plasmids. The wild type recombinant Factor VIII cDNA [pcDNA3.1(+) BDD-FVIII] expression construct is used. Mammalian cell line CHO is used as a heterologous protein expression system and is purchased from NCCS, Pune. CHO cells are cultured in DMEM-F12 medium containing 10% (v/v) fetal bovine serum (FBS) at 37°C in a 5% CO₂ incubator. Commercially available purified recombinant Factor VIII is purchased from Epitomics (France). Polyclonal antibodies against C2 domain of Factor VIII are raised in house. Restriction endonucleases are purchased from New England Biolabs. DMEM-F12 and FBS are purchased from Himedia and Lipofectamine 2000 Reagent is purchased from Invitrogen. Serum free media for CHO cell line is purchased from Sigma. Geniticin is purchased from Invitrogen and Pfu is purchased from Fermentas. Sepharose 4B coupled with Histidine-Bisoxirine is used. Coametric instruments are used for chromogenic assay.

### Example 2

### Construction of mutated recombinant Factor VIII

Site-directed mutagenesis of wild type recombinant Factor VIII cDNA (B domain deleted Factor VIII) is carried out. Mutations at F309S (Phe309Ser) and D519V (Asp519Val) are done using *pfu* polymerase with specific primers having desired mutation site integrated into it.

The pcDNA3.1(+) vector containing Factor VIII cDNA is used as template for site-directed mutagenesis. In an embodiment, the expression cassette is provided in Figure 4-
Vector (pcDNA3.1) - 5.4Kb
Insert (pcDNA-FVIII) - 4.6Kb
Total plasmid size - 10Kb

The specific primers are designed to mutate BDD-FVIII using the program PrimerX (http://www.bioinformatics.org/primerx/). The GC content and melting temperature is checked by MBCF (Molecular biology core facilities) oligo calculator (http://mbcf.dfci.harvard.edu/docs/ oligocalc.html). The PCR is performed using the following primers:

### Primers for F309S-

Forward primer (SEQ ID NO. 5): 5' CCTTGGACAGTTTCTACTGAGTTGTCATATCTCTTCCCAC 3'

Reverse primer (SEQ ID NO. 6): 5' GTGGGAAGAGATATGACAACTCAGTAGAAACTGTCCAAGG3'

### Primers for D519V-

Forward primer (SEQ ID NO. 7): 5' CAGTGACTGTAGAAGTTGGGCCA ACT AAATC 3'

Reverse primer (SEQ ID NO. 8): 5' GATTTAGTTGGCCCAACTTCTACAGTCACTG 3'

The specific PCR Programme cycling parameters are as follows-
a. Initial denaturation for 4 minutes (94°C) followed by 20 cycles of denaturation for 30 sec (94°C);
b. Annealing at 64.5°C for 45 sec;
c. Extension at 68°C for 3 min followed by a single cycle of Final extension at 72°C for 7 min.

The PCR amplicons obtained are further digested with DpnI and transformed into DH5α competent cells. The positive clones are selected and sequenced for mutation integration confirmation.

### Results

The double mutant recombinant Factor VIII cDNA [pcDNA3.1(+)ΔBDD-FVIII] expression construct having mutations at Phe309Ser and Asp519Val is generated by site directed mutagenesis. Mutation integration is confirmed by DNA sequencing.

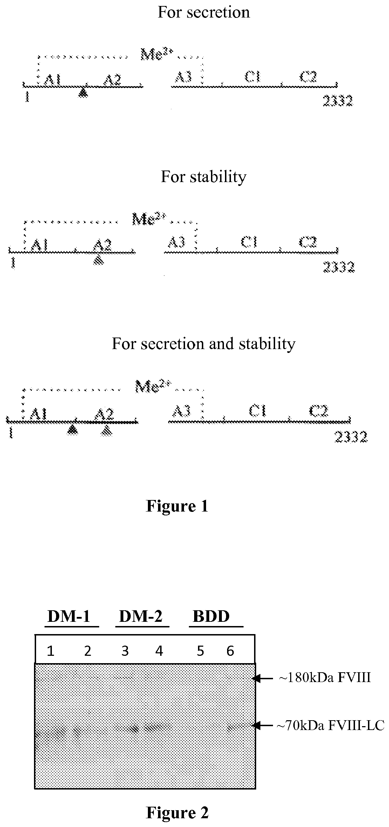

Figure 1 depicts the mutations carried out at the various sites of the BDD recombinant Factor VIII gene.

### Example 3

### Protein Expression studies

Mammalian cell line CHO is cultured in 10% FBS. Once the cells attain 70-80% confluency, antibiotic free media is added to the cells and then transfected with wild type recombinant Factor VIII cDNA [pcDNA3.1(+) BDDFVIII] expression construct and double mutant recombinant Factor VIII cDNA [pcDNA3.1(+)ΔBDD-FVIII] expression construct in independent experiments respectively. Transfection is carried out using Lipofectamine reagent. After 6 hours of transfection, optimal media is changed and complete media is added. 48 hours post transfection, antibiotic Geniticin is added to the plate for selecting stable clones. Single stable clones are picked up from the plate and are transferred to 96 well plate containing complete media with 560 µg/ml geniticin. Sufficient passaging is provided to the cells before culturing them in 75cm² flasks with 560 µg/ml geniticin. The cell culture supernatant is collected at various time points. For purification and further studies, stable clones are cultured in serum free media.

To analyze the expressed protein, Sodium Dodecyl Sulphate (SDS) poly acrylamide gel electrophoresis (PAGE) is carried out according to Laemmli and western blot is done to see the expression of the protein. Cell culture supernatant is mixed with 5X SDS sample loading buffer and boiled for 10 minutes. The samples are run on 8% SDS-PAGE gels at 80 volts for 120 minutes using Bio-Rad mini protein system. The resolved protein is transferred to nitrocellulose membrane at 90 volts 90 minutes in transfer buffer using Bio-Rad mini transblot apparatus. After transferring the protein to the nitrocellulose membrane, it is blocked with 5% skimmed milk powder. The membrane is then washed with PBS-T and incubated with anti C2 polyclonal antibody raised in-house for 2 hours and then with anti IgG conjugated with alkaline phosphatase. The positive reactivity is visualized using 5-bromo-4chloro-3-indolylphosphate/nitro blue tetrazonium (BCIP-NBT) as a substrate.

The aforementioned protein expression aspects are provided in a greater detail as follows:

### Materials:

### Media Requirements

EX-CELL® 325 PF CHO serum-free medium for CHO Cells without L-glutamine, protein free, liquid, sterile-filtered, suitable for cell culture is purchased from Sigma Aldrich, Bangalore. Fetal bovine serum (FBS) and DMEM-F12, are purchased from Himedia laboratories, India.

### Antibiotics and Reagents

Trypsin and Penicillin-streptomycin solution are purchased from Himedia laboratories, India. Geniticin is purchased from Invitrogen, LipofectamineTM2000 is purchased from Invitrogen. Other chemicals are obtained from Sigma-Aldrich (USA), SRL (India) and Merck Limited (India). Pfu DNA polymerase and dNTPs are obtained from Fermentas. T4 DNA ligase and restriction enzymes are purchased from New England Biolabs, U.S.A. Antibiotics Kanamycin and Ampicillin are from Himedia Lab, India. Kits used for plasmid isolation and gel extraction are from Sigma Aldrich, USA. Other chemicals are obtained from SRL (India) and Merck Limited (India). The oligonucleotide primers are synthesized at Sigma Aldrich Ltd (Bangalore, India).

### Culture Wares

Petri dishes are purchased from Tarsons, 96 well plates, 24 well plates, 12 well plates, 6 well plates, and 25cm² culture flasks are purchased from Nunclon, India. 75cm² culture flasks are purchased from Himedia Laboratories, India. Cryo vials are purchased from Tarsons.

### Instruments

The instruments used in the present disclosure are Phase contrast microscope (Olympus, USA), Centrifuge (Hettich Zentrifugen, Germany), Deionized water system (Milli Q, Millipore), Freezers -20°C and -80°C, CO₂ Incubator (Memmert, Germany), PCR machine (Eppendorf, Thermocycler model: 22331, Hamburg), Electrophoresis unit (Bio-Rad, USA), Gel documentation system (Bio rad, USA), Spectrophotometer (Beckman coulter, USA), Centrifuge (Eppendorf, Germany), Speed Vac, Deionized water system (Milli Q, Millipore), Ice machine, Incubators, Autoclave, Laminar air flow, Sonicator, pH meter, Water bath and minor lab equipments.

### Methods:

### Media Preparation

DMEM-F12 powder - 15 - 16 g; Autoclaved sterile water - 1L
Penicillin-Streptomycin solution - 1-2 ml
Sodium bicarbonate - 1 - 1.3 g
Milli Q water is autoclaved in a sterile flask. DMEM-F12 powder is added to sterile water and allowed to dissolve. 1.2g of sodium bicarbonate is added to the media. 2ml of penicillin-streptomycin antibiotics is added. pH of media is adjusted with NaOH or HCl. Media is filtered using 0.45µ filters. Sterility of the media is checked by overnight incubation at 37°C in incubator with 5% CO₂. 10% FBS is added to sterile media for adherent cell culture.

### PBS Preparation

NaCl - 137 mM
KCl - 2.7 mM
Na₂HPO₄ - 4.3 mM
KH₂PO₄ - 1.47 mM

The solution is dispensed into aliquots and sterilized by autoclaving. The solution is stored at room temperature between 22-24°C.

### Culturing Of CHO Cells:

CHO Cells are kept in liquid nitrogen until they are ready to be thawed. To thaw the cells, cryo-vials are placed in water bath at 37°C and cells are allowed to thaw. After homogenizing cells, it is transferred to a 15ml tarson tube with 9ml DMEM-F12 media supplemented with 10% FBS, centrifuged at 1500rpm for 5 minutes. The supernatant is discarded after centrifugation and pellet is suspended in 1ml of complete media and transferred it to 25cm² culture flasks with 5ml complete media. Culture flasks are placed in incubator at 37°C supplied with 5% CO₂. The cells are allowed to adhere to the surface. Cell growth is observed every 24 hours under phase contrast microscope. Once the cell reached 90-100% confluency, the media is discarded and passaged.

### TRANSFECTION:

### Requirements

CHO cell line
Lipofectamine
Plasmid DNA pcDNAFVIII mutated
DMEM-F12 media
FBS

### Procedure

One day before transfection, CHO cells are seeded onto 6 well plates. Each well has 0.5-2×10⁵ cells in 500µl growth medium without antibiotics. Prior to transfection, the complexes are prepared separately. 4µg of DNA is diluted in 50µl of optimum medium (media without FBS). The mixture is gently mixed. 10µl of lipofectamine 2000 is diluted in 50µl optimum medium and is incubated for 5 minutes. After 5 minutes incubation, diluted DNA and diluted Lipofectamine 2000 are combined (total volume 100µl). It is mixed gently and incubated for 20 minutes at room temperature. Medium is aspirated from plates and fresh medium is added without FBS and antibiotics before adding the complexes. 100µl of complexes are added to each well containing cells and medium. It is mixed gently so that the complex is distributed evenly and incubated at 37°C in CO₂ incubator. Complete medium is added after 6 hours of transfection. The cells are allowed to grow for 24 hours. Expression level is determined using GFP protein as control.

### Selection of Stable Clones Expressing mutated recombinant FVIII Protein (ΔBDD-FVIII) in CHO Cell Line:

### Requirements

DMEM-F12 media
Pen-strep solution
Trypsin
FBS
Geniticin

### Procedure

After transfection, cells are allowed to grow under non-selective conditions for 24 hours. After 24 hours, selection media is added with 560µg/ml of Geniticin. The cells are allowed to grow in selection media for 72 hours, after which fresh selection media is added. The clones are allowed to grow till it reached 90-100% confluency. Once the clones reached sufficient confluency, it is transferred to 96 well plate with same selection media. Clones are allowed to attach to surface and grow for 15 days or more. Passaging is done to 24 well plates, 12 well plates and 6 well plates with selection media.

### Selection of Single Clones Producing FVIII:

Once the stable clones are produced, single clones are selected from stable clones. Clones are selected from 12 well plates. The cells are trypsinized and stable clones are diluted in 10 ml selection media. Dilution is made after cell counting. 100µl of selection media statistically yields between 5-10 clones per 96 well plate, thereby minimizing the probability of wells with more than one clone. Cells are incubated under standard conditions and cells are feeded after 10-14 days with fresh selection medium. Clones are analyzed or further expanded as soon as cells in the non-transfected control wells have completely died. Once the resistant clones are identified, the cells are expanded and assayed for protein of interest by using appropriate analysis method.

### Culturing of Single Clones Expressing Mutated Factor VIII (ΔBDD-FVIII) in Serum Free Media:

CHO cell line is first cultured in DMEM-F12 supplemented with 10% FBS. Concentration of serum is decreased stepwise in every other passage. Cells are finally maintained in lower concentrations of FBS, up to 0.5%. Selected mutated single clones are cultured in DMEM-F12 supplied with 0.5% of FBS and after two passages they are transferred to serum free media. 3×10⁵ cells/ml are transferred to serum free media and allowed to grow at 37°C with 5% CO₂ supply. Cell culture supernatant is collected at various time points to check the protein production. Time points selected are 24, 48, 72, and 96 hours. Cell culture supernatant is then subjected to ELISA and Western blot for further analysis of secreted protein.

### Selection Of Stable Clones Expressing Mutated BDD-FVIII (ΔBDD-FVIII) By ELISA:

Stable CHO clones expressing mutated recombinant Factor VIII protein is analyzed by enzyme-linked immunosorbent assay (ELISA). Samples are collected from 96 well plate and coated onto ELISA plates for analysis. The basic principle of an ELISA is to use an enzyme to detect binding of antigen and antibody. Enzyme converts a colorless substrate to a colored product, indicating the presence of antigen: antibody complex.

### Requirements

Carbonate buffer/Bicarbonate buffer 0.1M, pH 9.6
NaHCO₃ - 8.4 g
Na₂CO₃ - 10.59 g
Milli Q water - 1L
pH adjusted to 9.6 and stored at room temperature
PBS pH 7.4
NaH₂PO₄ - 10 mM
Na₂HPO₄.2H₂O - 10 mm
NaCl - 150 mM
pH adjusted to 7.4

### Washing buffer

PBS - 1L
Tween 20 - 0.05%
Tween 20 added and mixed on a magnetic stirrer

### Dilution buffer

PBS - 10ml
Tween 20 - 0.05%
BSA - 0.5%
BSA added to PBS with Tween 20 solution.

### Blocking buffer

PBS - 10ml
BSA - 0.5%

### TMB solution

300mg of TMB is dissolved in 100 ml of DMSO. Stored in dark

### H₂O₂

3% of H₂O₂ is prepared

### Citrate/Phosphate buffer

Citric acid trisodium salt dihydrate - 29.4g
NaH₂PO₄.2H₂O - 17.19g
MilliQ water - 1L
The contents are added and pH adjusted to 5.0

### H₂SO₄

H₂SO₄ - 160 ml of concentrated H₂SO₄ is made up to 1L with MilliQ water

### Procedure

CHO stable clones expressing Factor VIII protein is collected from 96 well plate. 50µl of the sample is diluted in 0.1M bicarbonate buffer (pH 9.6), immobilized on 96 well ELISA plate for 20 hours at 4°C. Untransfected CHO is used as control. The plate is covered with aluminum foil. After 20 hours, plate is washed with washing solution, PBS and Tween 20. Remaining free space is blocked with BSA, incubating it for one hour at 37°C. It is then washed with washing buffer before adding primary antibody. Plate is incubated with polyclonal antibody raised against C2 domain of Factor VIII protein, with dilution of 1: 10,000. Antibody is diluted in dilution buffer, incubated for an hour at 37°C. Finally incubated with secondary antibody, Anti-IgG-rabbit-HRP conjugated (Sigma Aldrich, Bangalore), with a dilution of 1: 50,000 in dilution buffer. Incubation is done for an hour at 37°C. After washing thoroughly, 100µl of TMB solution is added to the wells, incubated for 5 minutes. Reaction is stopped by adding 50µl of H₂SO₄ (2M) to each well. Color change is noticed from blue to yellow. Reading is taken in an ELISA plate reader at 450nm.

### Protein Analysis by SDS-PAGE:

To analyze the expressed protein, SDS-PAGE is carried out according to Laemmli (Laemmli, 1970). Protein samples of 10 µl are mixed with 10 µl of 2x SDS sample loading buffer and boiled for 2 min. The samples are run on 10% SDS-PAGE gels at 100V for 90 min using Bio-Rad mini protein system (Bio-Rad Laboratories). The resolved protein samples are visualized by staining with Coomassie brilliant blue.

### Requirements

### Acrylamide/ Bis-acrylamide solution (30.8%)

Acrylamide - 30 gm
Bis-acrylamide - 0.8 gm
In 100 ml of double distilled water

### Resolving gel buffer: (pH 8.8)

Tris base - 18.18 gm in 70 ml of double distilled water
pH adjusted to 8.8 with conc. HCl
Water is added to make volume to 100 ml

### Stacking gel buffer (pH 6.8)

Tris base - 7.6 gm
In 100 ml double distilled water
pH adjusted to 6.8 with conc. HCl
25 ml double distilled water to make 125 ml

### Sample buffer

Stacking gel buffer - 5 ml
10% SDS - 8 ml
Glycerol - 4 ml
β-mercaptoethanol - 2 ml (added for reducing sample)
Bromo phenol blue indicator dye - 2 mg
The volume made upto 20 ml by adding distilled water
Ammonium per Sulphate (10%)
TEMED solution
Butanol

### Tank buffer (1X)

| | |
|---|---|
| Tris | - 6.06 mg |
| Glycine | - 28.8 mg |
| SDS | - 1 gm |
| In Double distilled water | - 1000 ml |

### Resolving gel composition (10%) (10 ml)

| | |
|---|---|
| Acrylamide/bisacrylamide solution (30.8%) | - 3.3 ml |
| Double distilled water | - 4.0 ml |
| 1.5M Tris (pH8.8) | - 2.5 ml |
| 10% SDS | - 0.1 ml |
| TEMED | - 004 ml |
| 10% APS | - 0.1 ml |

### Stacking gel composition (5%) (3 ml)

| | |
|---|---|
| Acrylamide/bisacrylamide solution (30.8%) | - 0.5 ml |
| Double distilled water | - 2.1 ml |
| 1.5 M Tris (pH 6.8) | - 0.38 ml |
| 10% SDS | - 0.03 ml |
| TEMED | - 0.003 ml |
| 10% APS | - 0.03 ml |

### Procedure

The two glass plates are assembled on a clean surface by keeping the longer glass plate containing spacers at the back and short thin plate in front. This glass plate sandwich is gently placed into the clamp assembly and tightened. This clamp assembly is placed on the casting stand, firmly against the rubber gasket at the base. The Resolving gel (8%) is prepared by mixing the appropriate concentration of the components gently, ensuring no air bubbles formation. The resolving gel is poured into the glass plate assembly. The gel is overlaid with butanol to ensure a flat surface and to exclude air. The butanol is washed off with water after the gel is set. Stacking gel (5%) is prepared and then poured on top of resolving gel. Comb (1 mm) is inserted gently and the gel is allowed to set. Then the comb is removed slowly. The gel assembly is placed in the buffer chamber and running buffer is added into the chamber. The sample is prepared by mixing the sample dye (2X) (reducing dye) with samples in the ratio of 1:1. Then the sample mixture is boiled for 5 min at 70°C, and centrifuged. 20 µl of the sample is loaded in the well and run at 100V for 90 min.

### Staining procedure: Silver Staining

### Requirements

### Solution I

Acetone - 30 ml
Water - 30 ml
Trichloro acetic acid - 0.75g

### Solution II

Acetone - 30 ml
Water - 30 ml

### Solution III

Water - 60 ml
Sodium thiosulphate 10% - 25µl
Formaldehyde - 25µl

### Solution IV

Silver nitrate - 0.16g
Water - 60ml

### Solution V

Water - 60 ml
Sodium carbonate - 1.2g
Formaldehyde - 100 µl

### Procedure

Gel is washed with water three times. The gel is fixed with Acetone and trichloro acetic acid for 5 minutes. After fixation, gel is washed with water three times. Then the gel is sensitized with sodium thiosulphate. After washing, gel is stained with silver nitrate by incubating in silver nitrate solution for 8 minutes. The gel is developed with developing solution, sodium carbonate. Once the bands are clear, the reaction is stopped by adding stop solution, glacial acetic acid. Gel is stored in water.

### Western Blotting

The expression of the recombinant protein is analyzed by transferring the protein from the SDS-PAGE gel to a nitrocellulose membrane and probing with anti Factor VIII polyclonal antibody raised in rabbit, in house.

### Requirements

### 10X PBS - 1 L

| | |
|---|---|
| NaCl | - 8g |
| KCl | - 0.2 g |
| Na₂HPO₄ | - 1.78 g |
| KH₂PO₄ | - 0.24 g |
| Distilled water | - Upto 1000 ml |
| pH | - 7.4 |

### Transfer Buffer- 1 L

| | |
|---|---|
| Tris | - 3.03 gm |
| Glycine | - 14.32 gm |
| Methanol | - 200 ml |
| Distilled water | - 800 ml |
| SDS (10%) | - 1 ml |

### Washing Buffer

| | |
|---|---|
| 1X PBS | - 1 L |
| Tween 20 | - 1 ml |

### Blocking buffer

| | |
|---|---|
| Skimmed milk powder - | - 5 gm |
| Tween 20 | - 100 µl |
| 1x PBS | - 100 ml |

Primary antibody - Anti Factor VIII antibody (polyclonal, raised in house)
Secondary antibody - Alkaline phosphatase conjugated antibody
Substrate - BCIP/NBT tablets (Sigma)

### Procedure

SDS-PAGE gel is run at 100V, according to the procedure explained in section SDS-PAGE procedure section, till the blue dye front reaches the bottom of the gel. To transfer proteins from gel to nitrocellulose membrane, materials used for transfer are soaked in transfer buffer for 15 min. Then they are stacked in the following order; case (clear side), sponge, Whatman paper, membrane, gel, Whatman paper, sponge case (black side) and placed in the transfer apparatus with black side facing black. The apparatus is immersed into ice filled bucket to cool the transfer buffer. Transfer is carried out at 90V for 90 min using Bio-Rad electro transfer apparatus. After transfer, the membrane is stained with 1X Ponceau S for a min, the bands marked with pencil, destained in water and rinsed in 1X PBS. Then the membrane is blocked for 1 h in 50 ml of 1X PBS + 5% non-fat dry milk + 0.1% Tween 20 on a shaker. Then the membrane is incubated with 1:3000 dilution of primary antibody in blocking buffer and incubated at RT for 1h or 4°C overnight. The membrane is incubated with alkaline phosphatase conjugated secondary antibody (1:10,000 dilutions) in blocking buffer for 1 h at room temperature. Between each step the membrane is washed three times with wash buffer containing PBS and 0.1% Tween 20 (PBS-T). One tablet, dissolved in 10 ml of water, provides 10 ml of ready to use buffered substrate solution. The substrate solution contains BCIP (0.15 mg/ml), NBT (0.30 mg/ml), Tris buffer (100 mM), and MgCl2 (5 mM), pH 9.25-9.75. The membrane is incubated in the substrate solution for 5 min.

### Results

Transfection of CHO cell lines are carried out. Selection of clones producing Factor VIII protein is done using geniticin 560µg/ml. Single clones are selected and maintained in 96 well plate, 48 well plate, 24 well plate, 6 well plate, 25cm flask and 75cm flask. Confirmation of the clones producing Factor VIII protein is done using ELISA (Table 1) and Western blot (Figure 2). The clone confirmation by western blot is done using C2 polyclonal antibody raised in house.

**Table 1**

| **S**.**NO** | **CELL LINE** | **ELISA VALUE** |
|---|---|---|
| 1 | CHO | 0.176 |
| 2 | BDD FVIII | 0.304 |
| 3 | | 0.289 |
| 4 | | **0**.**305** |
| 5 | | 0.207 |
| 6 | | **0**.**311** |
| 7 | ΔBDD FVIII | 0.237 |
| 8 | | 0.307 |
| 9 | | 0.116 |
| 10 | | **0**.**328** |
| 11 | | **0**.**316** |

This table 1 depicts ELISA confirmation of clones producing recombinant Protein. The cell culture media collected after 72 hrs is used in the experiment. CHO (control), B Domain Deleted Factor VIII i.e. wild-type Factor VIII without mutation (BDD-FVIII), Mutated B domain Deleted Factor VIII 309S+519V i.e. Double mutant recombinant Factor VIII with mutations at F309S and D519V (ΔBDD-FVIII-309S+519V).

Clones given in bold indicate the high protein secreting clones.

### Example 4

### Purification of recombinant Factor VIII

The protein of interest (wild type and double mutant recombinant Factor VIII) is purified using histidine ligand affinity chromatography (HLAC). The column is packed with 1mL of Histidine-Bisoxirine-Sepharose-4B gel. The column is equilibrated with binding buffer 20mM Tris pH 6.0. Cell culture supernatant containing the desired protein is loaded to the column after adjusting the pH of the supernatant to pH6.0 at a flow rate of 2ml per minute. After injecting the sample, all the non retained proteins are washed with the binding buffer till the baseline is achieved. The target protein is eluted with 20mM Tris containing 0.1M Glycine, 0.03M Lysine and 0.3M CaCl₂ at pH 7.0. The eluted peaks are concentrated using Amicon filters and analyzed on SDS-PAGE and the protein concentration is calculated by Bradford method. The purified fraction is confirmed by western blot analysis.

Even though HLAC has been used successfully in the purification of a number of recombinant and native proteins, high value therapeutic proteins with high molecular weight such as recombinant Factor VIII has not been reported to be purified using this technique. This is the first report which shows the purification of recombinant Factor VIII from cell culture supernatant using HLAC.

The aforementioned affinity purification procedure is provided in detail as follows:

### Requirements

Column type: Sepharose 4B
Colume volume: 1 ml
Load: 2 mg of protein
Equilibration/binding buffer: 20mM Tris pH6.0
Elution buffer: 20mM Tris + 0.1M Glycine + 0.03M Lysine + 0.3M CaCl₂ pH 7.0
Flow rate: 1 ml/min
Fraction volume: 2 ml

### Procedure

Cell culture supernatant containing BDD-FVIII and mutated BDD-FVIII protein (2 mg/ 50 ml) is loaded onto 1 ml Histidine-Bisoxirine-Sepharose 4B column, which is equilibrated with the equilibration/binding buffer at a flow rate of 1 ml/min. After injecting the sample, all the non retained proteins are washed with the binding buffer till the baseline is achieved. The target protein is eluted with elution buffer 20 mM Tris containing 0.1M Glycine, 0.03M Lysine and 0.3M CaCl₂. Protein concentration is determined by Bradford's assay. The purity of the eluted protein is analyzed by SDS-PAGE.

### Results

CHO cells are cultured in serum free media. Cell culture supernatant is collected after 48 hours. Cell culture supernatant containing mutated recombinant Factor VIII is subjected to affinity chromatography. Histidine-Bisoxirine-Sepharose-4B column is equilibrated with binding buffer 20mM Tris pH 6.0. The pH of cell culture supernatant is adjusted to 6.0 and directly injected to the column after filtration with 0.22µm filter. Flow through and elutions are collected for further analysis. The protein is eluted using 20mM Tris + 0.1M Glycine + 0.03M Lysine + 0.3M CaCl2 pH 7.0. The chromatogram shows a single peak (Figure 3A). SDS-PAGE analysis shows a clear band corresponding to full length FVIII (Figure 3B, Lane 5) which is confirmed by Western analsysis (Figure 5). The double arrows in Figure 3 correspond to fragments of FVIII which is also observed in purified commercial FVIII (Lane 6).

The heavy chain and light chain linkage of BDD-FVIII is stabilized only through metal ion. In the electrophoretic system, the electric current present will disrupt this linkage before the domain rearrangement happens. This explains the presence of the heavy chain and light chain separately due to the disruption in the linkage.

### Example 5

### Activity Assays

To confirm the presence of Factor VIII, one stage clotting assay and chromogenic assays are performed.

### One stage clotting assay

Factor VIII deficient plasma is dissolved in distilled or deionized water. Before use, it is allowed to stand for at least 15 minutes at 15 to 25°C, and then mixed carefully without foam formation. Wild type recombinant BDD Factor VIII and mutated BDD-FVIII sample produced in CHO cell lines are added to FVIII deficient plasma. APTT reagents are used according to the manufacturer's instructions. 0.025M of CaCl₂ is added to the mixture. The mixture is incubated at 37°C for 2 minutes and the reading is taken in an automated coagulation analyzer. Normal clotting time is 30-40 seconds.

### Chromogenic assay

For the photometric determination of Factor VIII activity, chromogenic assay is done. In the presence of Calcium and phospholipids, factor x is activated to factor Xa by factor IXa. This generation is stimulated by Factor VIII. The rate of activation of factor X solely depends on the amount of Factor VIII. Factor IXa hydrolyses the chromogenic substrate S-2765, thus liberating the chromophoric group, pNA. The color is then read photometrically at 405nm. The generated factor Xa and thus the intensity of the color are directly proportional to the Factor VIII activity in the sample. The said chromogenic assay is further detailed as follows.

Appropriate amount of BDD-FVIII and mutated BDD-FVIII sample is taken in plastic test tubes. Specific controls for plasma or Factor VIII concentrates is calibrated against the international standard of Factor VIII. The detection limit is 0.05IU/mL and low range of 0.005IU/mL is detected in case of low range.

After reconstituting the Factor (Factor VIII, Factor IX, and Factor X) reagents, chromogenic substrate along with inhibitor is reconstituted and then the buffer is prepared.

### Results

The activity assays are carried out for double mutant BDD-FVIII Samples and wild type BDD-FVIII Samples. The purified Factor VIII samples are added to Factor VIII depleted plasma and one stage clotting assay is done (Table 2). Chromogenic assay result for wild type BDD-FVIII Samples and double mutant BDD-FVIII Samples reveal that the double mutant BDD-FVIII protein shows about 3.7 fold increase in activity compared to wild type BDD-FVIII protein (Table 2).

The one stage clotting assay and chromogenic assay was carried out for BDD-FVIII type, single and double mutant of the present invention and the results are tabulated below.

**Table 2: One stage clotting assay results [the experiments are repeated thrice and the mean values are represented]**

| **Name** | **% FVIII Activity** | **Specific Activity (IU/mg)** |
|---|---|---|
| BDD-FVIII | 0.795 | 2053 |
| BDD-FVIII (single mutant-Phe309Ser) | 0.823 | 4064 |
| BDD-FVIII (single mutant-Asp519Val) | 0.0091 | 24.82 |
| BDD-FVIII (double mutant) | 0.900 | 7978 |

### INFERENCE:

The Factor VIII double mutant has higher specific activity (3.88 fold) when compared to wild type BDD FVIII as determined by one stage clotting assay. The single mutant BDD-FVIII (Phe309Ser) shows approximately 2 fold increase when compared to the activity of the wild type BDD FVIII, whereas the single mutant BDD-FVIII (Asp519Val) had negligible activity.

**Table 3: Chromogenic assay results**

| **Name** | **% of Factor VIII activity** | **Units/Amount of Protein [Specific Activity]** |
|---|---|---|
| BDD-FVIII | 0.419 | 1082 |
| BDD-FVIII (single mutant-Phe309Ser) | 0.432 | 2133 |
| BDD-FVIII (single mutant-Asp519Val) | 0.0009 | 2.482 |
| BDD-FVIII (double mutant) | 0.455 | 4033 |

### INFERENCE:

The Factor VIII double mutant has higher specific activity (3.727 fold) when compared to BDD FVIII as determined by chromogenic assay. The single mutant BDD-FVIII (Phe309Ser) shows approximately 2 fold increase when compared to the activity of the wild type BDD FVIII, whereas the single mutant BDD-FVIII (Asp519Val) had negligible activity.

### CONCLUSION:

Both the one stage clotting assay and chromogenic assay results showed that there is synergistic effect shown by the double mutant which is reflected in terms of enhanced activity over the wild type BDD FVIII and any of the single mutant BDD-FVIII.

### Example 6

### Addition of divalent metal ion to the purified Factor VIII protein and Activity Assays

The purified wild type and double mutant Factor VIII samples are incubated with 5mM CaCl₂ for 20 hours and the specific activity is measured using chromogenic assay.

### Results

Addition of divalent metal ion to the purified wild type and mutated Factor VIII protein results in an increased specific activity of the protein. The result of chromogenic assay indicates that there is a 6 fold increase in the specific activity of double mutant Factor VIII compared to wild type Factor VIII when CaCl₂ is added to both wild type and double mutant proteins and incubated for 20 hours (Table 4).

**Table 4: One stage clotting assay results after the addition of divalent metal ion**

| **Name** | **%FVIII activity** | **Specific activity (Units/Amount of protein) [Specific Activity]** |
|---|---|---|
| BDD-FVIII | 0.832 | 2148 |
| BDD-FVIII-F309S | 0.82 | 4049 |
| BDD-FVIII-D519V | 1.5 | 4092 |
| BDD-FVIII-F309S+D519V | 1.46 | 12943 |

### Example 8

### One stage clotting assay results of 10th, 20th and 30th generation of the clones of wild type and the double mutant FVIII

The one stage clotting assay results of the 10^{th} generation, 20^{th} generation and the 30^{th} generation of the clones of wild type BDD-FVIII was noted. The one stage clotting assay results of the 10^{th} generation, 20^{th} generation and the 30^{th} generation of the clones of the double mutant BDD-FVIII-F309S+D519V is tabulated in Table 6.

### Results

The results from the below table (Table 6) show the stability of % FVIII activity and the specific activity of the mutated factor VIII when compared to wild type factor VIII over 10^{th}, 20^{th} and the 30^{th} generations.

**Table 6: One stage clotting assay results of 10th, 20th and 30th generation of the clones of wild type and the double mutant FVIII**

| **Name** | **% FVIII activity** | **Specific activity (IU/mg)** |
|---|---|---|
| BDD-FVIII (10^{th} Generation) | 0.216 | 2160 |
| BDD-FVIII (20^{th} Generation) | 0.207 | 2070 |
| BDD-FVIII (30^{th} Generation) | 0.244 | 2440 |
| BDD-FVIII-F309S+D519V (10^{th} Generation) | 0.874 | 7748 |
| BDD-FVIII-F309S+D519V (20^{th} Generation) | 0.85 | 7727 |
| BDD-FVIII-F309S+D519V (30^{th} Generation) | 0.88 | 7927 |

## Claims

1. A Factor VIII represented by the amino acid sequence as set forth in SEQ ID NO. 14.

2. The Factor VIII of claim 1, wherein the sequence corresponds to mature B-Domain Deleted (BDD) FVIII double mutant polypeptide comprising the mutations F309S and D519V.

3. A nucleic acid represented by the nucleotide sequence as set forth in SEQ ID No. 13.

4. The nucleic acid of claim 3, wherein the sequence corresponds to the B-domain deleted Factor VIII gene and comprises mutations at positions 925-927 and 1555-1557 of said nucleotide sequence; wherein the mutation in the nucleotide sequence at position 925-927 corresponds to the replacement of phenylalanine with serine at position 309 in the corresponding amino acid sequence and the mutation in the nucleotide sequence at position 1555-1557 corresponds to the replacement of aspartic acid with valine at position 519 in the corresponding amino acid sequence; and wherein the mutations are point mutations at positions 925, 926 and 1556 of said nucleotide sequence.

5. An expression cassette or a vector having an expression cassette comprising the nucleic acid of claim 3.

6. The expression cassette or the vector of claim 5, wherein the expression cassette or the vector comprises the nucleic acid represented by SEQ ID NO. 13 along with a signal peptide coding sequence; and wherein said vector is inserted into *E.coli* deposited under accession number MTCC 5855.

7. A host cell comprising the expression cassette or the vector of claim 5.

8. A method of obtaining the nucleic acid as set forth in SEQ ID No. 13 along with a signal peptide coding sequence by mutating the nucleic acid as set forth in SEQ ID No.3 at positions 925-927 and 1555-1557, calculated after the signal peptide coding sequence of 1-57 nucleotides.

9. A method of obtaining a transformed host cell comprising an expression cassette as claimed in claim 6, said method comprising
a. inserting the expression cassette as claimed in claim 6 into a vector; and
b. transforming the host cell with said vector to obtain the transformed host cell.

10. A method of producing a protein having the amino acid sequence of claim 1, said method comprising:
a. mutating positions 925-927 and 1555-1557 calculated after the signal peptide coding sequence of 1-57 nucleotides of the nucleic acid sequence as set forth in SEQ ID No.3 to obtain a nucleic acid as set forth in SEQ ID NO.13 along with a signal peptide coding sequence;
b. transforming a host cell comprising a vector having an expression cassette comprising the nucleotide sequence set forth as SEQ ID No. 13 along with a signal peptide coding sequence; and
c. culturing the transformed host cell in a culture medium for producing said protein.

11. The method of claim 8 or 10 wherein the nucleotide sequence as set forth in SEQ ID No.3 corresponds to the wild-type B-domain deleted Factor VIII gene; wherein the mutation at position 925-927 corresponds to the replacement of phenylalanine with serine at position 309 in the amino acid sequence and the mutation at position 1555-1557 corresponds to the replacement of aspartic acid with valine at position 519 in the amino acid sequence.

12. A kit or a composition comprising a protein having the amino acid sequence of claim 1 optionally along with excipients.

13. An in vitro method of activating factor X/enhancing plasma deficient in factor VIII, said method comprises incubating factor X or a sample comprising factor X/plasma deficient in factor VIII with mature recombinant double mutant factor VIII protein having the amino acid sequence as claimed in claim 1.

14. A composition comprising a protein having the amino acid sequence as set forth in SEQ ID NO. 14 optionally along with a pharmaceutically acceptable carrier or excipient or a combination thereof, for use in a method of managing coagulation disorder(s).

## Patentansprüche

1. Faktor VIII, dargestellt durch die Aminosäuresequenz wie dargelegt in SEQ ID NR. 14.

2. Faktor VIII von Anspruch 1, wobei die Sequenz reifem B-Domäne deletiertem (BDD) FVIII Doppelmutanten-Polypeptid entspricht, umfassend die Mutationen F309S und D519V.

3. Nukleinsäure, dargestellt durch die Nukleotidsequenz, wie dargelegt in SEQ ID Nr. 13.

4. Nukleinsäure von Anspruch 3, wobei die Sequenz dem B-Domäne deletiertem -Faktor VIII-Gen entspricht und Mutationen an Positionen 925-927 und 1555-1557 der Nukleotidsequenz umfasst; wobei die Mutation in der Nukleotidsequenz an Position 925-927 dem Austausch von Phenylalanin mit Serin an Position 309 in der entsprechenden Aminosäuresequenz entspricht und die Mutation in der Nukleotidsequenz an Position 1555-1557 dem Austausch von Asparaginsäure mit Valin an Position 519 in der entsprechenden Aminosäuresequenz entspricht; und wobei die Mutationen Punktmutationen an Positionen 925, 926 und 1556 der Nukleotidsequenz sind.

5. Expressionskassette oder Vektor mit einer Expressionskassette, umfassend die Nukleinsäure von Anspruch 3.

6. Expressionskassette oder Vektor von Anspruch 5, wobei die Expressionskassette oder der Vektor die Nukleinsäure, dargestellt durch SEQ ID NR. 13, zusammen mit einer Signalpeptid kodierenden Sequenz umfasst; und wobei der Vektor in *E.coli,* hinterlegt unter Hinterlegungsnummer MTCC 5855, eingefügt ist.

7. Wirtszelle, umfassend die Expressionskassette oder den Vektor von Anspruch 5.

8. Verfahren zum Erhalten der Nukleinsäure, wie dargelegt in SEQ ID Nr. 13, zusammen mit einer Signalpeptid kodierenden Sequenz durch Mutieren der Nukleinsäure, wie dargelegt in SEQ ID Nr. 3, an Positionen 925-927 und 1555-1557, berechnet nach der Signalpeptid kodierenden Sequenz von 1-57 Nukleotiden.

9. Verfahren zum Erhalten einer transformierten Wirtszelle, umfassend eine Expressionskassette wie in Anspruch 6 beansprucht, das Verfahren umfassend:
a. Einfügen der Expressionskassette, wie in Anspruch 6 beansprucht, in einen Vektor; und
b. Transformieren der Wirtszelle mit dem Vektor, um die transformierte Wirtszelle zu erhalten.

10. Verfahren zur Herstellung eines Proteins mit der Aminosäuresequenz von Anspruch 1, das Verfahren umfassend:
a. Mutieren von Positionen 925-927 und 1555-1557, berechnet nach der Signalpeptid kodierenden Sequenz von 1-57 Nukleotiden der Nukleinsäuresequenz, wie dargelegt in SEQ ID Nr. 3, um eine Nukleinsäure, wie dargelegt in SEQ ID NR. 13, zusammen mit einer Signalpeptid kodierenden Sequenz zu erhalten;
b. Transformieren einer Wirtszelle, umfassend einen Vektor mit einer Expressionskassette, umfassend die Nukleotidsequenz, wie dargelegt als SEQ ID Nr. 13, zusammen mit einer Signalpeptid kodierenden Sequenz; und
c. Kultivieren der transformierten Wirtszelle in einem Kulturmedium zur Herstellung des Proteins.

11. Verfahren nach Anspruch 8 oder 10, wobei die Nukleotidsequenz, wie dargelegt in SEQ ID Nr. 3, dem Wildtyp-B-Domäne deletierten-Faktor VIII-Gen entspricht; wobei die Mutation an Position 925-927 dem Austausch von Phenylalanin mit Serin an Position 309 in der Aminosäuresequenz entspricht und die Mutation an Position 1555-1557 dem Austausch von Asparaginsäure mit Valin an Position 519 in der Aminosäuresequenz entspricht.

12. Kit oder Zusammensetzung, umfassend ein Protein mit der Aminosäuresequenz von Anspruch 1, optional zusammen mit Exzipienten.

13. In-vitro-Verfahren zur Aktivierung von Faktor X/ Verbesserung von Plasma, mangelnd an Faktor VIII, wobei das Verfahren Inkubieren von Faktor X oder eine Probe, umfassend Faktor X/Plasma, mangelnd an Faktor VIII, mit reifem rekombinantem Doppelmutanten-Faktor VIII-Protein mit der Aminosäuresequenz, wie in Anspruch 1 beansprucht, umfasst.

14. Zusammensetzung, umfassend ein Protein mit der Aminosäuresequenz, wie dargelegt in SEQ ID NR. 14, optional zusammen mit einem pharmazeutisch unbedenklichen Träger oder Exzipienten oder einer Kombination davon, zur Verwendung in einem Verfahren zum Handhaben von Koagulationsstörung(en).

## Revendications

1. Facteur VIII représenté par la séquence d'acides aminés telle qu'indiquée par SEQ ID NO : 14.

2. Facteur VIII selon la revendication 1, dans lequel la séquence correspond au polypeptide mature double mutant du FVIII délété du domaine B (BDD) comprenant les mutations F309S et D519V.

3. Acide nucléique représenté par la séquence nucléotidique telle qu'indiquée par SEQ ID NO : 13.

4. Acide nucléique selon la revendication 3, dans lequel la séquence correspond au gène du facteur VIII délété du domaine B et comprend des mutations au niveau des positions 925 à 927 et 1555 à 1557 de ladite séquence nucléotidique ; dans lequel la mutation dans la séquence nucléotidique niveau de la position 925 à 927 correspond au remplacement d'une phénylalanine par une sérine au niveau de la position 309 dans la séquence d'acides aminés correspondante et la mutation dans la séquence nucléotidique au niveau de la position 1555 à 1557 correspond au remplacement d'un acide aspartique par une valine au niveau de la position 519 dans la séquence d'acides aminés correspondante ; et dans lequel les mutations sont des mutations ponctuelles au niveau des positions 925, 926 et 1556 de ladite séquence nucléotidique.

5. Cassette d'expression ou vecteur comportant une cassette d'expression comprenant l'acide nucléique selon la revendication 3.

6. Cassette d'expression ou vecteur selon la revendication 5, dans laquelle/lequel la cassette d'expression ou le vecteur comprend l'acide nucléique représenté par SEQ ID NO : 13 accompagné d'une séquence codante de peptide signal ; et dans lequel ledit vecteur est inséré dans *E. coli* déposé sous le numéro d'accession MTCC 5855.

7. Cellule hôte comprenant la cassette d'expression ou le vecteur selon la revendication 5.

8. Procédé d'obtention de l'acide nucléique tel qu'indiqué par SEQ ID NO : 13 accompagné d'une séquence codante de peptide signal par mutation de l'acide nucléique tel qu'indiqué par SEQ ID NO : 3 au niveau des positions 925 à 927 et 1555 à 1557, calculées après la séquence codante de peptide signal de nucléotides 1 à 57.

9. Procédé d'obtention d'une cellule hôte transformée comprenant une cassette d'expression selon la revendication 6, ledit procédé comprenant :
a. l'insertion de la cassette d'expression selon la revendication 6 dans un vecteur ; et
b. la transformation de la cellule hôte avec ledit vecteur pour obtenir la cellule hôte transformée.

10. Procédé de production d'une protéine ayant la séquence d'acides aminés selon la revendication 1, ledit procédé comprenant :
a. la mutation des positions 925 à 927 et 1555 à 1557 calculées après la séquence codante de peptide signal de nucléotides 1 à 57 de la séquence d'acide nucléique telle qu'indiquée par SEQ ID NO : 3 pour obtenir un acide nucléique tel qu'indiqué par SEQ ID NO : 13 accompagné d'une séquence codante de peptide signal ;
b. la transformation d'une cellule hôte comprenant le vecteur comportant une cassette d'expression comprenant la séquence nucléotidique telle qu'indiquée par SEQ ID NO : 13 accompagnée d'une séquence codante de peptide signal ; et
c. la culture de la cellule hôte transformée dans un milieu de culture pour produire ladite protéine.

11. Procédé selon la revendication 8 ou 10 dans lequel la séquence nucléotidique telle qu'indiquée par SEQ ID NO : 3 correspond au gène de type sauvage du facteur VIII délété du domaine B ; dans lequel la mutation au niveau de la position 925 à 927 correspond au remplacement d'une phénylalanine par une sérine au niveau de la position 309 dans la séquence d'acides aminés et la mutation au niveau de la position 1555 à 1557 correspond au remplacement d'un acide aspartique par une valine au niveau de la position 519 dans la séquence d'acides aminés.

12. Kit ou composition comprenant une protéine ayant la séquence d'acides aminés selon la revendication 1 éventuellement accompagnée d'excipients.

13. Procédé *in vitro* d'activation du facteur X/d'amélioration du plasma déficient en facteur VIII, ledit procédé comprenant l'incubation de facteur X ou d'un échantillon comprenant du facteur X/du plasma déficient en facteur VIII l avec de la protéine mature recombinante double mutante du facteur VIII ayant la séquence d'acides aminés selon la revendication 1.

14. Composition comprenant une protéine ayant la séquence d'acides aminés telle qu'indiquée par SEQ ID NO : 14 éventuellement accompagnée d'un support ou d'un excipient pharmaceutiquement acceptable ou de l'une de leurs combinaisons, pour une utilisation dans un procédé de prise en charge d'un ou de plusieurs troubles de la coagulation.


### SEQUENCE LISTING

<110> Centre for Bioseparation Technology; CHRISTIAN MEDICAL COLLEGE and Department Of Biotechnology
<120> DOUBLE MUTANT COAGULATION FACTOR VIII, METHODS AND APPLICATIONS THEREOF
<130> IP20619/SUFY
<160> 14
<170> PatentIn version 3.5
<210> 1
   <211> 4377
   <212> DNA
   <213> Homo sapiens
<220>
   <221> gene
   <222> (1)..(4377)
<400> 1
   atgcaaatag agctctccac ctgcttcttt ctgtgccttt tgcgattctg ctttagtgcc      60
   accagaagat actacctggg tgcagtggaa ctgtcatggg actatatgca aagtgatctc     120
   ggtgagctgc ctgtggacgc aagatttcct cctagagtgc caaaatcttt tccattcaac     180
   acctcagtcg tgtacaaaaa gactctgttt gtagaattca cggatcacct tttcaacatc     240
   gctaagccaa ggccaccctg gatgggtctg ctaggtccta ccatccaggc tgaggtttat     300
   gatacagtgg tcattacact taagaacatg gcttcccatc ctgtcagtct tcatgctgtt     360
   ggtgtatcct actggaaagc ttctgaggga gctgaatatg atgatcagac cagtcaaagg     420
   gagaaagaag atgataaagt cttccctggt ggaagccata catatgtctg gcaggtcctg     480
   aaagagaatg gtccaatggc ctctgaccca ctgtgcctta cctactcata tctttctcat     540
   gtggacctgg taaaagactt gaattcaggc ctcattggag ccctactagt atgtagagaa     600
   gggagtctgg ccaaggaaaa gacacagacc ttgcacaaat ttatactact ttttgctgta     660
   tttgatgaag ggaaaagttg gcactcagaa acaaagaact ccttgatgca ggatagggat     720
   gctgcatctg ctcgggcctg gcctaaaatg cacacagtca atggttatgt aaacaggtct     780
   ctgccaggtc tgattggatg ccacaggaaa tcagtctatt ggcatgtgat tggaatgggc     840
   accactcctg aagtgcactc aatattcctc gaaggtcaca catttcttgt gaggaaccat     900
   cgccaggcgt ccttggaaat ctcgccaata actttcctta ctgctcaaac actcttgatg     960
   gaccttggac agtttctact gagttgtcat atctcttccc accaacatga tggcatggaa    1020
   gcttatgtca aagtagacag ctgtccagag gaaccccaac tacgaatgaa aaataatgaa    1080
   gaagcggaag actatgatga tgatcttact gattctgaaa tggatgtggt caggtttgat    1140
   gatgacaact ctccttcctt tatccaaatt cgctcagttg ccaagaagca tcctaaaact 1200
   tgggtacatt acattgctgc tgaagaggag gactgggact atgctccctt agtcctcgcc 1260
   cccgatgaca gaagttataa aagtcaatat ttgaacaatg gccctcagcg gattggtagg 1320
   aagtacaaaa aagtccgatt tatggcatac acagatgaaa cctttaagac tcgtgaagct 1380
   attcagcatg aatcaggaat cttgggacct ttactttatg gggaagttgg agacacactg 1440
   ttgattatat ttaagaatca agcaagcaga ccatataaca tctaccctca cggaatcact 1500
   gatgtccgtc ctttgtattc aaggagatta ccaaaaggtg taaaacattt gaaggatttt 1560
   ccaattctgc caggagaaat attcaaatat aaatggacag tgactgtaga agttgggcca 1620
   actaaatcag atcctcggtg cctgacccgc tattactcta gtttcgttaa tatggagaga 1680
   gatctagctt caggactcat tggccctctc ctcatctgct acaaagaatc tgtagatcaa 1740
   agaggaaacc agataatgtc agacaagagg aatgtcatcc tgttttctgt atttgatgag 1800
   aaccgaagct ggtacctcac agagaatata caacgctttc tccccaatcc agctggagtg 1860
   cagcttgagg atccagagtt ccaagcctcc aacatcatgc acagcatcaa tggctatgtt 1920
   tttgatagtt tgcagttgtc agtttgtttg catgaggtgg catactggta cattctaagc 1980
   attggagcac agactgactt cctttctgtc ttcttctctg gatatacctt caaacacaaa 2040
   atggtctatg aagacacact caccctattc ccattctcag gagaaactgt cttcatgtcg 2100
   atggaaaacc caggtctatg gattctgggg tgccacaact cagactttcg gaacagaggc 2160
   atgaccgcct tactgaaggt ttctagttgt gacaagaaca ctggtgatta ttacgaggac 2220
   agttatgaag atatttcagc atacttgctg agtaaaaaca atgccattga accaagaagc 2280
   ttctcccagc aaaacccacc agtcttgaaa cgccatcaac gggaaataac tcgtactact 2340
   cttcagtcag atcaagagga aattgactat gatgatacca tatcagttga aatgaagaag 2400
   gaagattttg acatttatga tgaggatgaa aatcagagcc cccgcagctt tcaaaagaaa 2460
   acacgacact attttattgc tgcagtggag aggctctggg attatgggat gagtagctcc 2520
   ccacatgttc taagaaacag ggctcagagt ggcagtgtcc ctcagttcaa gaaagttgtt 2580
   ttccaggaat ttactgatgg ctcctttact cagcccttat accgtggaga actaaatgaa 2640
   catttgggac tcctggggcc atatataaga gcagaagttg aagataatat catggtaact 2700
   ttcagaaatc aggcctctcg tccctattcc ttctattcta gccttatttc ttatgaggaa 2760
   gatcagaggc aaggagcaga acctagaaaa aactttgtca agcctaatga aaccaaaact 2820
   tacttttgga aagtgcaaca tcatatggca cccactaaag atgagtttga ctgcaaagcc 2880
   tgggcttatt tctctgatgt tgacctggaa aaagatgtgc actcaggcct gattggaccc 2940
   cttctggtct gccacactaa cacactgaac cctgctcatg ggagacaagt gacagtacag 3000
   gaatttgctc tgtttttcac catctttgat gagaccaaaa gctggtactt cactgaaaat 3060
   atggaaagaa actgcagggc tccctgcaat atccagatgg aagatcccac ttttaaagag 3120
   aattatcgct tccatgcaat caatggctac ataatggata cactacctgg cttagtaatg 3180
   gctcaggatc aaaggattcg atggtatctg ctcagcatgg gcagcaatga aaacatccat 3240
   tctattcatt tcagtggaca tgtgttcact gtacgaaaaa aagaggagta taaaatggca 3300
   ctgtacaatc tctatccagg tgtttttgag acagtggaaa tgttaccatc caaagctgga 3360
   atttggcggg tggaatgcct tattggcgag catctacatg ctgggatgag cacacttttt 3420
   ctggtgtaca gcaataagtg tcagactccc ctgggaatgg cttctggaca cattagagat 3480
   tttcagatta cagcttcagg acaatatgga cagtgggccc caaagctggc cagacttcat 3540
   tattccggat caatcaatgc ctggagcacc aaggagccct tttcttggat caaggtggat 3600
   ctgttggcac caatgattat tcacggcatc aagacccagg gtgcccgtca gaagttctcc 3660
   agcctctaca tctctcagtt tatcatcatg tatagtcttg atgggaagaa gtggcagact 3720
   tatcgaggaa attccactgg aaccttaatg gtcttctttg gcaatgtgga ttcatctggg 3780
   ataaaacaca atatttttaa ccctccaatt attgctcgat acatccgttt gcacccaact 3840
   cattatagca ttcgcagcac tcttcgcatg gagttgatgg gctgtgattt aaatagttgc 3900
   agcatgccat tgggaatgga gagtaaagca atatcagatg cacagattac tgcttcatcc 3960
   tactttacca atatgtttgc cacctggtct ccttcaaaag ctcgacttca cctccaaggg 4020
   aggagtaatg cctggagacc tcaggtgaat aatccaaaag agtggctgca agtggacttc 4080
   cagaagacaa tgaaagtcac aggagtaact actcagggag taaaatctct gcttaccagc 4140
   atgtatgtga aggagttcct catctccagc agtcaagatg gccatcagtg gactctcttt 4200
   tttcagaatg gcaaagtaaa ggtttttcag ggaaatcaag actccttcac acctgtggtg 4260
   aactctctag acccaccgtt actgactcgc taccttcgaa ttcaccccca gagttgggtg 4320
   caccagattg ccctgaggat ggaggttctg ggctgcgagg cacaggacct ctactga 4377
<210> 2
   <211> 1458
   <212> PRT
   <213> Homo sapiens
<220>
   <221> PEPTIDE
   <222> (1)..(1458)
<400> 2
   Met Gln Ile Glu Leu Ser Thr Cys Phe Phe Leu Cys Leu Leu Arg Phe
   1               5                   10                  15
   Cys Phe Ser Ala Thr Arg Arg Tyr Tyr Leu Gly Ala Val Glu Leu Ser
               20                  25                  30
   Trp Asp Tyr Met Gln Ser Asp Leu Gly Glu Leu Pro Val Asp Ala Arg
           35                  40                  45
   Phe Pro Pro Arg Val Pro Lys Ser Phe Pro Phe Asn Thr Ser Val Val
       50                  55                  60
   Tyr Lys Lys Thr Leu Phe Val Glu Phe Thr Asp His Leu Phe Asn Ile
   65                  70                  75                  80
   Ala Lys Pro Arg Pro Pro Trp Met Gly Leu Leu Gly Pro Thr Ile Gln
                   85                  90                  95
   Ala Glu Val Tyr Asp Thr Val Val Ile Thr Leu Lys Asn Met Ala Ser
               100                 105                 110
   His Pro Val Ser Leu His Ala Val Gly Val Ser Tyr Trp Lys Ala Ser
           115                 120                 125
   Glu Gly Ala Glu Tyr Asp Asp Gln Thr Ser Gln Arg Glu Lys Glu Asp
       130                 135                 140
   Asp Lys Val Phe Pro Gly Gly Ser His Thr Tyr Val Trp Gln Val Leu
   145                 150                 155                 160
   Lys Glu Asn Gly Pro Met Ala Ser Asp Pro Leu Cys Leu Thr Tyr Ser
                   165                 170                 175
   Tyr Leu Ser His Val Asp Leu Val Lys Asp Leu Asn Ser Gly Leu Ile
               180                 185                 190
   Gly Ala Leu Leu Val Cys Arg Glu Gly Ser Leu Ala Lys Glu Lys Thr
           195                 200                 205
   Gln Thr Leu His Lys Phe Ile Leu Leu Phe Ala Val Phe Asp Glu Gly
       210                 215                 220
   Lys Ser Trp His Ser Glu Thr Lys Asn Ser Leu Met Gln Asp Arg Asp
   225                 230                 235                 240
   Ala Ala Ser Ala Arg Ala Trp Pro Lys Met His Thr Val Asn Gly Tyr
                   245                 250                 255
   Val Asn Arg Ser Leu Pro Gly Leu Ile Gly Cys His Arg Lys Ser Val
               260                 265                 270
   Tyr Trp His Val Ile Gly Met Gly Thr Thr Pro Glu Val His Ser Ile
           275                 280                 285
   Phe Leu Glu Gly His Thr Phe Leu Val Arg Asn His Arg Gln Ala Ser
       290                 295                 300
   Leu Glu Ile Ser Pro Ile Thr Phe Leu Thr Ala Gln Thr Leu Leu Met
   305                 310                 315                 320
   Asp Leu Gly Gln Phe Leu Leu Ser Cys His Ile Ser Ser His Gln His
                   325                 330                 335
   Asp Gly Met Glu Ala Tyr Val Lys Val Asp Ser Cys Pro Glu Glu Pro
               340                 345                 350
   Gln Leu Arg Met Lys Asn Asn Glu Glu Ala Glu Asp Tyr Asp Asp Asp
           355                 360                 365
   Leu Thr Asp Ser Glu Met Asp Val Val Arg Phe Asp Asp Asp Asn Ser
       370                 375                 380
   Pro Ser Phe Ile Gln Ile Arg Ser Val Ala Lys Lys His Pro Lys Thr
   385                 390                 395                 400
   Trp Val His Tyr Ile Ala Ala Glu Glu Glu Asp Trp Asp Tyr Ala Pro
                   405                 410                 415
   Leu Val Leu Ala Pro Asp Asp Arg Ser Tyr Lys Ser Gln Tyr Leu Asn
               420                 425                 430
   Asn Gly Pro Gln Arg Ile Gly Arg Lys Tyr Lys Lys Val Arg Phe Met
           435                 440                 445
   Ala Tyr Thr Asp Glu Thr Phe Lys Thr Arg Glu Ala Ile Gln His Glu
       450                 455                 460
   Ser Gly Ile Leu Gly Pro Leu Leu Tyr Gly Glu Val Gly Asp Thr Leu
   465                 470                 475                 480
   Leu Ile Ile Phe Lys Asn Gln Ala Ser Arg Pro Tyr Asn Ile Tyr Pro
                   485                 490                 495
   His Gly Ile Thr Asp Val Arg Pro Leu Tyr Ser Arg Arg Leu Pro Lys
               500                 505                 510
   Gly Val Lys His Leu Lys Asp Phe Pro Ile Leu Pro Gly Glu Ile Phe
           515                 520                 525
   Lys Tyr Lys Trp Thr Val Thr Val Glu Val Gly Pro Thr Lys Ser Asp
       530                 535                 540
   Pro Arg Cys Leu Thr Arg Tyr Tyr Ser Ser Phe Val Asn Met Glu Arg
   545                 550                 555                 560
   Asp Leu Ala Ser Gly Leu Ile Gly Pro Leu Leu Ile Cys Tyr Lys Glu
                   565                 570                 575
   Ser Val Asp Gln Arg Gly Asn Gln Ile Met Ser Asp Lys Arg Asn Val
               580                 585                 590
   Ile Leu Phe Ser Val Phe Asp Glu Asn Arg Ser Trp Tyr Leu Thr Glu
           595                 600                 605
   Asn Ile Gln Arg Phe Leu Pro Asn Pro Ala Gly Val Gln Leu Glu Asp
       610                 615                 620
   Pro Glu Phe Gln Ala Ser Asn Ile Met His Ser Ile Asn Gly Tyr Val
   625                 630                 635                 640
   Phe Asp Ser Leu Gln Leu Ser Val Cys Leu His Glu Val Ala Tyr Trp
                   645                 650                 655
   Tyr Ile Leu Ser Ile Gly Ala Gln Thr Asp Phe Leu Ser Val Phe Phe
               660                 665                 670
   Ser Gly Tyr Thr Phe Lys His Lys Met Val Tyr Glu Asp Thr Leu Thr
           675                 680                 685
   Leu Phe Pro Phe Ser Gly Glu Thr Val Phe Met Ser Met Glu Asn Pro
       690                 695                 700
   Gly Leu Trp Ile Leu Gly Cys His Asn Ser Asp Phe Arg Asn Arg Gly
   705                 710                 715                 720
   Met Thr Ala Leu Leu Lys Val Ser Ser Cys Asp Lys Asn Thr Gly Asp
                   725                 730                 735
   Tyr Tyr Glu Asp Ser Tyr Glu Asp Ile Ser Ala Tyr Leu Leu Ser Lys
               740                 745                 750
   Asn Asn Ala Ile Glu Pro Arg Ser Phe Ser Gln Gln Asn Pro Pro Val
           755                 760                 765
   Leu Lys Arg His Gln Arg Glu Ile Thr Arg Thr Thr Leu Gln Ser Asp
       770                 775                 780
   Gln Glu Glu Ile Asp Tyr Asp Asp Thr Ile Ser Val Glu Met Lys Lys
   785                 790                 795                 800
   Glu Asp Phe Asp Ile Tyr Asp Glu Asp Glu Asn Gln Ser Pro Arg Ser
                   805                 810                 815
   Phe Gln Lys Lys Thr Arg His Tyr Phe Ile Ala Ala Val Glu Arg Leu
               820                 825                 830
   Trp Asp Tyr Gly Met Ser Ser Ser Pro His Val Leu Arg Asn Arg Ala
           835                 840                 845
   Gln Ser Gly Ser Val Pro Gln Phe Lys Lys Val Val Phe Gln Glu Phe
       850                 855                 860
   Thr Asp Gly Ser Phe Thr Gln Pro Leu Tyr Arg Gly Glu Leu Asn Glu
   865                 870                 875                 880
   His Leu Gly Leu Leu Gly Pro Tyr Ile Arg Ala Glu Val Glu Asp Asn
                   885                 890                 895
   Ile Met Val Thr Phe Arg Asn Gln Ala Ser Arg Pro Tyr Ser Phe Tyr
               900                 905                 910
   Ser Ser Leu Ile Ser Tyr Glu Glu Asp Gln Arg Gln Gly Ala Glu Pro
           915                 920                 925
   Arg Lys Asn Phe Val Lys Pro Asn Glu Thr Lys Thr Tyr Phe Trp Lys
       930                 935                 940
   Val Gln His His Met Ala Pro Thr Lys Asp Glu Phe Asp Cys Lys Ala
   945                 950                 955                 960
   Trp Ala Tyr Phe Ser Asp Val Asp Leu Glu Lys Asp Val His Ser Gly
                   965                 970                 975
   Leu Ile Gly Pro Leu Leu Val Cys His Thr Asn Thr Leu Asn Pro Ala
               980                 985                 990
   His Gly Arg Gln Val Thr Val Gln  Glu Phe Ala Leu Phe  Phe Thr Ile
           995                 1000                1005
   Phe Asp  Glu Thr Lys Ser Trp  Tyr Phe Thr Glu Asn  Met Glu Arg
       1010                1015                1020
   Asn Cys  Arg Ala Pro Cys Asn  Ile Gln Met Glu Asp  Pro Thr Phe
       1025                1030                1035
   Lys Glu  Asn Tyr Arg Phe His  Ala Ile Asn Gly Tyr  Ile Met Asp
       1040                1045                1050
   Thr Leu  Pro Gly Leu Val Met  Ala Gln Asp Gln Arg  Ile Arg Trp
       1055                1060                1065
   Tyr Leu  Leu Ser Met Gly Ser  Asn Glu Asn Ile His  Ser Ile His
       1070                1075                1080
   Phe Ser  Gly His Val Phe Thr  Val Arg Lys Lys Glu  Glu Tyr Lys
       1085                1090                1095
   Met Ala  Leu Tyr Asn Leu Tyr  Pro Gly Val Phe Glu  Thr Val Glu
       1100                1105                1110
   Met Leu  Pro Ser Lys Ala Gly  Ile Trp Arg Val Glu  Cys Leu Ile
       1115                1120                1125
   Gly Glu  His Leu His Ala Gly  Met Ser Thr Leu Phe  Leu Val Tyr
       1130                1135                1140
   Ser Asn  Lys Cys Gln Thr Pro  Leu Gly Met Ala Ser  Gly His Ile
       1145                1150                1155
   Arg Asp  Phe Gln Ile Thr Ala  Ser Gly Gln Tyr Gly  Gln Trp Ala
       1160                1165                1170
   Pro Lys  Leu Ala Arg Leu His  Tyr Ser Gly Ser Ile  Asn Ala Trp
       1175                1180                1185
   Ser Thr  Lys Glu Pro Phe Ser  Trp Ile Lys Val Asp  Leu Leu Ala
       1190                1195                1200
   Pro Met  Ile Ile His Gly Ile  Lys Thr Gln Gly Ala  Arg Gln Lys
       1205                1210                1215
   Phe Ser  Ser Leu Tyr Ile Ser  Gln Phe Ile Ile Met  Tyr Ser Leu
       1220                1225                1230
   Asp Gly  Lys Lys Trp Gln Thr  Tyr Arg Gly Asn Ser  Thr Gly Thr
       1235                1240                1245
   Leu Met  Val Phe Phe Gly Asn  Val Asp Ser Ser Gly  Ile Lys His
       1250                1255                1260
   Asn Ile Phe Asn Pro Pro Ile Ile Ala Arg Tyr Ile Arg Leu His
   1265 1270 1275
   Pro Thr His Tyr Ser Ile Arg Ser Thr Leu Arg Met Glu Leu Met
   1280 1285 1290
   Gly Cys Asp Leu Asn Ser Cys Ser Met Pro Leu Gly Met Glu Ser
   1295 1300 1305
   Lys Ala Ile Ser Asp Ala Gln Ile Thr Ala Ser Ser Tyr Phe Thr
   1310 1315 1320
   Asn Met Phe Ala Thr Trp Ser Pro Ser Lys Ala Arg Leu His Leu
   1325 1330 1335
   Gln Gly Arg Ser Asn Ala Trp Arg Pro Gln Val Asn Asn Pro Lys
   1340 1345 1350
   Glu Trp Leu Gln Val Asp Phe Gln Lys Thr Met Lys Val Thr Gly
   1355 1360 1365
   Val Thr Thr Gln Gly Val Lys Ser Leu Leu Thr Ser Met Tyr Val
   1370 1375 1380
   Lys Glu Phe Leu Ile Ser Ser Ser Gln Asp Gly His Gln Trp Thr
   1385 1390 1395
   Leu Phe Phe Gln Asn Gly Lys Val Lys Val Phe Gln Gly Asn Gln
   1400 1405 1410
   Asp Ser Phe Thr Pro Val Val Asn Ser Leu Asp Pro Pro Leu Leu
   1415 1420 1425
   Thr Arg Tyr Leu Arg Ile His Pro Gln Ser Trp Val His Gln Ile
   1430 1435 1440
   Ala Leu Arg Met Glu Val Leu Gly Cys Glu Ala Gln Asp Leu Tyr
   1445 1450 1455
<210> 3
   <211> 4377
   <212> DNA
   <213> Homo sapiens
<220>
   <221> gene
   <222> (1)..(4377)
<400> 3
   atgcaaatag agctctccac ctgcttcttt ctgtgccttt tgcgattctg ctttagtgcc      60
   accagaagat actacctggg tgcagtggaa ctgtcatggg actatatgca aagtgatctc     120
   ggtgagctgc ctgtggacgc aagatttcct cctagagtgc caaaatcttt tccattcaac     180
   acctcagtcg tgtacaaaaa gactctgttt gtagaattca cggatcacct tttcaacatc     240
   gctaagccaa ggccaccctg gatgggtctg ctaggtccta ccatccaggc tgaggtttat     300
   gatacagtgg tcattacact taagaacatg gcttcccatc ctgtcagtct tcatgctgtt     360
   ggtgtatcct actggaaagc ttctgaggga gctgaatatg atgatcagac cagtcaaagg     420
   gagaaagaag atgataaagt cttccctggt ggaagccata catatgtctg gcaggtcctg     480
   aaagagaatg gtccaatggc ctctgaccca ctgtgcctta cctactcata tctttctcat     540
   gtggacctgg taaaagactt gaattcaggc ctcattggag ccctactagt atgtagagaa     600
   gggagtctgg ccaaggaaaa gacacagacc ttgcacaaat ttatactact ttttgctgta      660
   tttgatgaag ggaaaagttg gcactcagaa acaaagaact ccttgatgca ggatagggat     720
   gctgcatctg ctcgggcctg gcctaaaatg cacacagtca atggttatgt aaacaggtct     780
   ctgccaggtc tgattggatg ccacaggaaa tcagtctatt ggcatgtgat tggaatgggc     840
   accactcctg aagtgcactc aatattcctc gaaggtcaca catttcttgt gaggaaccat     900
   cgccaggcgt ccttggaaat ctcgccaata actttcctta ctgctcaaac actcttgatg     960
   gaccttggac agtttctact gttttgtcat atctcttccc accaacatga tggcatggaa    1020
   gcttatgtca aagtagacag ctgtccagag gaaccccaac tacgaatgaa aaataatgaa    1080
   gaagcggaag actatgatga tgatcttact gattctgaaa tggatgtggt caggtttgat    1140
   gatgacaact ctccttcctt tatccaaatt cgctcagttg ccaagaagca tcctaaaact    1200
   tgggtacatt acattgctgc tgaagaggag gactgggact atgctccctt agtcctcgcc    1260
   cccgatgaca gaagttataa aagtcaatat ttgaacaatg gccctcagcg gattggtagg    1320
   aagtacaaaa aagtccgatt tatggcatac acagatgaaa cctttaagac tcgtgaagct    1380
   attcagcatg aatcaggaat cttgggacct ttactttatg gggaagttgg agacacactg    1440
   ttgattatat ttaagaatca agcaagcaga ccatataaca tctaccctca cggaatcact    1500
   gatgtccgtc ctttgtattc aaggagatta ccaaaaggtg taaaacattt gaaggatttt    1560
   ccaattctgc caggagaaat attcaaatat aaatggacag tgactgtaga agatgggcca    1620
   actaaatcag atcctcggtg cctgacccgc tattactcta gtttcgttaa tatggagaga    1680
   gatctagctt caggactcat tggccctctc ctcatctgct acaaagaatc tgtagatcaa    1740
   agaggaaacc agataatgtc agacaagagg aatgtcatcc tgttttctgt atttgatgag    1800
   aaccgaagct ggtacctcac agagaatata caacgctttc tccccaatcc agctggagtg    1860
   cagcttgagg atccagagtt ccaagcctcc aacatcatgc acagcatcaa tggctatgtt    1920
   tttgatagtt tgcagttgtc agtttgtttg catgaggtgg catactggta cattctaagc 1980
   attggagcac agactgactt cctttctgtc ttcttctctg gatatacctt caaacacaaa 2040
   atggtctatg aagacacact caccctattc ccattctcag gagaaactgt cttcatgtcg 2100
   atggaaaacc caggtctatg gattctgggg tgccacaact cagactttcg gaacagaggc 2160
   atgaccgcct tactgaaggt ttctagttgt gacaagaaca ctggtgatta ttacgaggac 2220
   agttatgaag atatttcagc atacttgctg agtaaaaaca atgccattga accaagaagc 2280
   ttctcccagc aaaacccacc agtcttgaaa cgccatcaac gggaaataac tcgtactact 2340
   cttcagtcag atcaagagga aattgactat gatgatacca tatcagttga aatgaagaag 2400
   gaagattttg acatttatga tgaggatgaa aatcagagcc cccgcagctt tcaaaagaaa 2460
   acacgacact attttattgc tgcagtggag aggctctggg attatgggat gagtagctcc 2520
   ccacatgttc taagaaacag ggctcagagt ggcagtgtcc ctcagttcaa gaaagttgtt 2580
   ttccaggaat ttactgatgg ctcctttact cagcccttat accgtggaga actaaatgaa 2640
   catttgggac tcctggggcc atatataaga gcagaagttg aagataatat catggtaact 2700
   ttcagaaatc aggcctctcg tccctattcc ttctattcta gccttatttc ttatgaggaa 2760
   gatcagaggc aaggagcaga acctagaaaa aactttgtca agcctaatga aaccaaaact 2820
   tacttttgga aagtgcaaca tcatatggca cccactaaag atgagtttga ctgcaaagcc 2880
   tgggcttatt tctctgatgt tgacctggaa aaagatgtgc actcaggcct gattggaccc 2940
   cttctggtct gccacactaa cacactgaac cctgctcatg ggagacaagt gacagtacag 3000
   gaatttgctc tgtttttcac catctttgat gagaccaaaa gctggtactt cactgaaaat 3060
   atggaaagaa actgcagggc tccctgcaat atccagatgg aagatcccac ttttaaagag 3120
   aattatcgct tccatgcaat caatggctac ataatggata cactacctgg cttagtaatg 3180
   gctcaggatc aaaggattcg atggtatctg ctcagcatgg gcagcaatga aaacatccat 3240
   tctattcatt tcagtggaca tgtgttcact gtacgaaaaa aagaggagta taaaatggca 3300
   ctgtacaatc tctatccagg tgtttttgag acagtggaaa tgttaccatc caaagctgga 3360
   atttggcggg tggaatgcct tattggcgag catctacatg ctgggatgag cacacttttt 3420
   ctggtgtaca gcaataagtg tcagactccc ctgggaatgg cttctggaca cattagagat 3480
   tttcagatta cagcttcagg acaatatgga cagtgggccc caaagctggc cagacttcat 3540
   tattccggat caatcaatgc ctggagcacc aaggagccct tttcttggat caaggtggat 3600
   ctgttggcac caatgattat tcacggcatc aagacccagg gtgcccgtca gaagttctcc 3660
   agcctctaca tctctcagtt tatcatcatg tatagtcttg atgggaagaa gtggcagact 3720
   tatcgaggaa attccactgg aaccttaatg gtcttctttg gcaatgtgga ttcatctggg 3780
   ataaaacaca atatttttaa ccctccaatt attgctcgat acatccgttt gcacccaact     3840
   cattatagca ttcgcagcac tcttcgcatg gagttgatgg gctgtgattt aaatagttgc     3900
   agcatgccat tgggaatgga gagtaaagca atatcagatg cacagattac tgcttcatcc     3960
   tactttacca atatgtttgc cacctggtct ccttcaaaag ctcgacttca cctccaaggg     4020
   aggagtaatg cctggagacc tcaggtgaat aatccaaaag agtggctgca agtggacttc     4080
   cagaagacaa tgaaagtcac aggagtaact actcagggag taaaatctct gcttaccagc     4140
   atgtatgtga aggagttcct catctccagc agtcaagatg gccatcagtg gactctcttt     4200
   tttcagaatg gcaaagtaaa ggtttttcag ggaaatcaag actccttcac acctgtggtg     4260
   aactctctag acccaccgtt actgactcgc taccttcgaa ttcaccccca gagttgggtg     4320
   caccagattg ccctgaggat ggaggttctg ggctgcgagg cacaggacct ctactga         4377
<210> 4
   <211> 1458
   <212> PRT
   <213> Homo sapiens
<220>
   <221> PEPTIDE
   <222> (1)..(1458)
<400> 4
   Met Gln Ile Glu Leu Ser Thr Cys Phe Phe Leu Cys Leu Leu Arg Phe
   1               5                   10                  15
   Cys Phe Ser Ala Thr Arg Arg Tyr Tyr Leu Gly Ala Val Glu Leu Ser
               20                  25                  30
   Trp Asp Tyr Met Gln Ser Asp Leu Gly Glu Leu Pro Val Asp Ala Arg
           35                  40                  45
   Phe Pro Pro Arg Val Pro Lys Ser Phe Pro Phe Asn Thr Ser Val Val
       50                  55                  60
   Tyr Lys Lys Thr Leu Phe Val Glu Phe Thr Asp His Leu Phe Asn Ile
   65                  70                  75                  80
   Ala Lys Pro Arg Pro Pro Trp Met Gly Leu Leu Gly Pro Thr Ile Gln
                   85                  90                  95
   Ala Glu Val Tyr Asp Thr Val Val Ile Thr Leu Lys Asn Met Ala Ser
               100                 105                 110
   His Pro Val Ser Leu His Ala Val Gly Val Ser Tyr Trp Lys Ala Ser
           115                 120                 125
   Glu Gly Ala Glu Tyr Asp Asp Gln Thr Ser Gln Arg Glu Lys Glu Asp
       130                 135                 140
   Asp Lys Val Phe Pro Gly Gly Ser His Thr Tyr Val Trp Gln Val Leu
   145                 150                 155                 160
   Lys Glu Asn Gly Pro Met Ala Ser Asp Pro Leu Cys Leu Thr Tyr Ser
                   165                 170                 175
   Tyr Leu Ser His Val Asp Leu Val Lys Asp Leu Asn Ser Gly Leu Ile
               180                 185                 190
   Gly Ala Leu Leu Val Cys Arg Glu Gly Ser Leu Ala Lys Glu Lys Thr
           195                 200                 205
   Gln Thr Leu His Lys Phe Ile Leu Leu Phe Ala Val Phe Asp Glu Gly
       210                 215                 220
   Lys Ser Trp His Ser Glu Thr Lys Asn Ser Leu Met Gln Asp Arg Asp
   225                 230                 235                 240
   Ala Ala Ser Ala Arg Ala Trp Pro Lys Met His Thr Val Asn Gly Tyr
                   245                 250                 255
   Val Asn Arg Ser Leu Pro Gly Leu Ile Gly Cys His Arg Lys Ser Val
               260                 265                 270
   Tyr Trp His Val Ile Gly Met Gly Thr Thr Pro Glu Val His Ser Ile
           275                 280                 285
   Phe Leu Glu Gly His Thr Phe Leu Val Arg Asn His Arg Gln Ala Ser
       290                 295                 300
   Leu Glu Ile Ser Pro Ile Thr Phe Leu Thr Ala Gln Thr Leu Leu Met
   305                 310                 315                 320
   Asp Leu Gly Gln Phe Leu Leu Phe Cys His Ile Ser Ser His Gln His
                   325                 330                 335
   Asp Gly Met Glu Ala Tyr Val Lys Val Asp Ser Cys Pro Glu Glu Pro
               340                 345                 350
   Gln Leu Arg Met Lys Asn Asn Glu Glu Ala Glu Asp Tyr Asp Asp Asp
           355                 360                 365
   Leu Thr Asp Ser Glu Met Asp Val Val Arg Phe Asp Asp Asp Asn Ser
   370 375 380
   Pro Ser Phe Ile Gln Ile Arg Ser Val Ala Lys Lys His Pro Lys Thr
   385 390 395 400
   Trp Val His Tyr Ile Ala Ala Glu Glu Glu Asp Trp Asp Tyr Ala Pro
   405 410 415
   Leu Val Leu Ala Pro Asp Asp Arg Ser Tyr Lys Ser Gln Tyr Leu Asn
   420 425 430
   Asn Gly Pro Gln Arg Ile Gly Arg Lys Tyr Lys Lys Val Arg Phe Met
   435 440 445
   Ala Tyr Thr Asp Glu Thr Phe Lys Thr Arg Glu Ala Ile Gln His Glu
   450 455 460
   Ser Gly Ile Leu Gly Pro Leu Leu Tyr Gly Glu Val Gly Asp Thr Leu
   465 470 475 480
   Leu Ile Ile Phe Lys Asn Gln Ala Ser Arg Pro Tyr Asn Ile Tyr Pro
   485 490 495
   His Gly Ile Thr Asp Val Arg Pro Leu Tyr Ser Arg Arg Leu Pro Lys
   500 505 510
   Gly Val Lys His Leu Lys Asp Phe Pro Ile Leu Pro Gly Glu Ile Phe
   515 520 525
   Lys Tyr Lys Trp Thr Val Thr Val Glu Asp Gly Pro Thr Lys Ser Asp
   530 535 540
   Pro Arg Cys Leu Thr Arg Tyr Tyr Ser Ser Phe Val Asn Met Glu Arg
   545 550 555 560
   Asp Leu Ala Ser Gly Leu Ile Gly Pro Leu Leu Ile Cys Tyr Lys Glu
   565 570 575
   Ser Val Asp Gln Arg Gly Asn Gln Ile Met Ser Asp Lys Arg Asn Val
   580 585 590
   Ile Leu Phe Ser Val Phe Asp Glu Asn Arg Ser Trp Tyr Leu Thr Glu
   595 600 605
   Asn Ile Gln Arg Phe Leu Pro Asn Pro Ala Gly Val Gln Leu Glu Asp
   610 615 620
   Pro Glu Phe Gln Ala Ser Asn Ile Met His Ser Ile Asn Gly Tyr Val
   625                 630                 635                 640
   Phe Asp Ser Leu Gln Leu Ser Val Cys Leu His Glu Val Ala Tyr Trp
                   645                 650                 655
   Tyr Ile Leu Ser Ile Gly Ala Gln Thr Asp Phe Leu Ser Val Phe Phe
               660                 665                 670
   Ser Gly Tyr Thr Phe Lys His Lys Met Val Tyr Glu Asp Thr Leu Thr
           675                 680                 685
   Leu Phe Pro Phe Ser Gly Glu Thr Val Phe Met Ser Met Glu Asn Pro
       690                 695                 700
   Gly Leu Trp Ile Leu Gly Cys His Asn Ser Asp Phe Arg Asn Arg Gly
   705                 710                 715                 720
   Met Thr Ala Leu Leu Lys Val Ser Ser Cys Asp Lys Asn Thr Gly Asp
                   725                 730                 735
   Tyr Tyr Glu Asp Ser Tyr Glu Asp Ile Ser Ala Tyr Leu Leu Ser Lys
               740                 745                 750
   Asn Asn Ala Ile Glu Pro Arg Ser Phe Ser Gln Gln Asn Pro Pro Val
           755                 760                 765
   Leu Lys Arg His Gln Arg Glu Ile Thr Arg Thr Thr Leu Gln Ser Asp
       770                 775                 780
   Gln Glu Glu Ile Asp Tyr Asp Asp Thr Ile Ser Val Glu Met Lys Lys
   785                 790                 795                 800
   Glu Asp Phe Asp Ile Tyr Asp Glu Asp Glu Asn Gln Ser Pro Arg Ser
                   805                 810                 815
   Phe Gln Lys Lys Thr Arg His Tyr Phe Ile Ala Ala Val Glu Arg Leu
               820                 825                 830
   Trp Asp Tyr Gly Met Ser Ser Ser Pro His Val Leu Arg Asn Arg Ala
           835                 840                 845
   Gln Ser Gly Ser Val Pro Gln Phe Lys Lys Val Val Phe Gln Glu Phe
       850                 855                 860
   Thr Asp Gly Ser Phe Thr Gln Pro Leu Tyr Arg Gly Glu Leu Asn Glu
   865                 870                 875                 880
   His Leu Gly Leu Leu Gly Pro Tyr Ile Arg Ala Glu Val Glu Asp Asn
                   885                 890                 895
   Ile Met Val Thr Phe Arg Asn Gln Ala Ser Arg Pro Tyr Ser Phe Tyr
               900                 905                 910
   Ser Ser Leu Ile Ser Tyr Glu Glu Asp Gln Arg Gln Gly Ala Glu Pro
           915                 920                 925
   Arg Lys Asn Phe Val Lys Pro Asn Glu Thr Lys Thr Tyr Phe Trp Lys
       930                 935                 940
   Val Gln His His Met Ala Pro Thr Lys Asp Glu Phe Asp Cys Lys Ala
   945                 950                 955                 960
   Trp Ala Tyr Phe Ser Asp Val Asp Leu Glu Lys Asp Val His Ser Gly
                   965                 970                 975
   Leu Ile Gly Pro Leu Leu Val Cys His Thr Asn Thr Leu Asn Pro Ala
               980                 985                 990
   His Gly Arg Gln Val Thr Val Gln  Glu Phe Ala Leu Phe  Phe Thr Ile
           995                 1000                 1005
   Phe Asp  Glu Thr Lys Ser Trp  Tyr Phe Thr Glu Asn  Met Glu Arg
       1010                 1015                 1020
   Asn Cys  Arg Ala Pro Cys Asn  Ile Gln Met Glu Asp  Pro Thr Phe
       1025                 1030                 1035
   Lys Glu  Asn Tyr Arg Phe His  Ala Ile Asn Gly Tyr  Ile Met Asp
       1040                 1045                 1050
   Thr Leu  Pro Gly Leu Val Met  Ala Gln Asp Gln Arg  Ile Arg Trp
       1055                 1060                 1065
   Tyr Leu  Leu Ser Met Gly Ser  Asn Glu Asn Ile His  Ser Ile His
       1070                 1075                 1080
   Phe Ser  Gly His Val Phe Thr  Val Arg Lys Lys Glu  Glu Tyr Lys
       1085                 1090                 1095
   Met Ala  Leu Tyr Asn Leu Tyr  Pro Gly Val Phe Glu  Thr Val Glu
       1100                 1105                 1110
   Met Leu  Pro Ser Lys Ala Gly  Ile Trp Arg Val Glu  Cys Leu Ile
       1115                 1120                 1125
   Gly Glu His Leu His Ala Gly Met Ser Thr Leu Phe Leu Val Tyr
       1130                1135                1140
   Ser Asn Lys Cys Gln Thr Pro Leu Gly Met Ala Ser Gly His Ile
       1145                1150                1155
   Arg Asp Phe Gln Ile Thr Ala Ser Gly Gln Tyr Gly Gln Trp Ala
       1160                1165                1170
   Pro Lys Leu Ala Arg Leu His Tyr Ser Gly Ser Ile Asn Ala Trp
       1175                1180                1185
   Ser Thr Lys Glu Pro Phe Ser Trp Ile Lys Val Asp Leu Leu Ala
       1190                1195                1200
   Pro Met Ile Ile His Gly Ile Lys Thr Gln Gly Ala Arg Gln Lys
       1205                1210                1215
   Phe Ser Ser Leu Tyr Ile Ser Gln Phe Ile Ile Met Tyr Ser Leu
       1220                1225                1230
   Asp Gly Lys Lys Trp Gln Thr Tyr Arg Gly Asn Ser Thr Gly Thr
       1235                1240                1245
   Leu Met Val Phe Phe Gly Asn Val Asp Ser Ser Gly Ile Lys His
       1250                1255                1260
   Asn Ile Phe Asn Pro Pro Ile Ile Ala Arg Tyr Ile Arg Leu His
       1265                1270                1275
   Pro Thr His Tyr Ser Ile Arg Ser Thr Leu Arg Met Glu Leu Met
       1280                1285                1290
   Gly Cys Asp Leu Asn Ser Cys Ser Met Pro Leu Gly Met Glu Ser
       1295                1300                1305
   Lys Ala Ile Ser Asp Ala Gln Ile Thr Ala Ser Ser Tyr Phe Thr
       1310                1315                1320
   Asn Met Phe Ala Thr Trp Ser Pro Ser Lys Ala Arg Leu His Leu
       1325                1330                1335
   Gln Gly Arg Ser Asn Ala Trp Arg Pro Gln Val Asn Asn Pro Lys
       1340                1345                1350
   Glu Trp Leu Gln Val Asp Phe Gln Lys Thr Met Lys Val Thr Gly
       1355                1360                1365
   Val Thr  Thr Gln Gly Val Lys  Ser Leu Leu Thr Ser  Met Tyr Val
       1370                1375                1380
   Lys Glu  Phe Leu Ile Ser Ser  Ser Gln Asp Gly His  Gln Trp Thr
       1385                1390                1395
   Leu Phe  Phe Gln Asn Gly Lys  Val Lys Val Phe Gln  Gly Asn Gln
       1400                1405                1410
   Asp Ser  Phe Thr Pro Val Val  Asn Ser Leu Asp Pro  Pro Leu Leu
       1415                1420                1425
   Thr Arg  Tyr Leu Arg Ile His  Pro Gln Ser Trp Val  His Gln Ile
       1430                1435                1440
   Ala Leu  Arg Met Glu Val Leu  Gly Cys Glu Ala Gln  Asp Leu Tyr
       1445                1450                1455
<210> 5
   <211> 40
   <212> DNA
   <213> synthetic sequence
<220>
   <221> primer_bind
   <222> (1)..(40)
<400> 5
   ccttggacag tttctactga gttgtcatat ctcttcccac 40
<210> 6
   <211> 40
   <212> DNA
   <213> synthetic sequence
<220>
   <221> primer_bind
   <222> (1)..(40)
<400> 6
   gtgggaagag atatgacaac tcagtagaaa ctgtccaagg 40
<210> 7
   <211> 31
   <212> DNA
   <213> synthetic sequence
<220>
   <221> primer_bind
   <222> (1)..(31)
<400> 7
   cagtgactgt agaagttggg ccaactaaat c 31
<210> 8
   <211> 31
   <212> DNA
   <213> synthetic sequence
<220>
   <221> primer_bind
   <222> (1)..(31)
<400> 8
   gatttagttg gcccaacttc tacagtcact g 31
<210> 9
   <211> 4377
   <212> DNA
   <213> Homo sapiens
<220>
   <221> gene
   <222> (1)..(4377)
<400> 9
   atgcaaatag agctctccac ctgcttcttt ctgtgccttt tgcgattctg ctttagtgcc   60
   accagaagat actacctggg tgcagtggaa ctgtcatggg actatatgca aagtgatctc  120
   ggtgagctgc ctgtggacgc aagatttcct cctagagtgc caaaatcttt tccattcaac  180
   acctcagtcg tgtacaaaaa gactctgttt gtagaattca cggatcacct tttcaacatc  240
   gctaagccaa ggccaccctg gatgggtctg ctaggtccta ccatccaggc tgaggtttat  300
   gatacagtgg tcattacact taagaacatg gcttcccatc ctgtcagtct tcatgctgtt  360
   ggtgtatcct actggaaagc ttctgaggga gctgaatatg atgatcagac cagtcaaagg  420
   gagaaagaag atgataaagt cttccctggt ggaagccata catatgtctg gcaggtcctg  480
   aaagagaatg gtccaatggc ctctgaccca ctgtgcctta cctactcata tctttctcat  540
   gtggacctgg taaaagactt gaattcaggc ctcattggag ccctactagt atgtagagaa  600
   gggagtctgg ccaaggaaaa gacacagacc ttgcacaaat ttatactact ttttgctgta   660
   tttgatgaag ggaaaagttg gcactcagaa acaaagaact ccttgatgca ggatagggat  720
   gctgcatctg ctcgggcctg gcctaaaatg cacacagtca atggttatgt aaacaggtct  780
   ctgccaggtc tgattggatg ccacaggaaa tcagtctatt ggcatgtgat tggaatgggc  840
   accactcctg aagtgcactc aatattcctc gaaggtcaca catttcttgt gaggaaccat  900
   cgccaggcgt ccttggaaat ctcgccaata actttcctta ctgctcaaac actcttgatg  960
   gaccttggac agtttctact gagttgtcat atctcttccc accaacatga tggcatggaa 1020
   gcttatgtca aagtagacag ctgtccagag gaaccccaac tacgaatgaa aaataatgaa 1080
   gaagcggaag actatgatga tgatcttact gattctgaaa tggatgtggt caggtttgat 1140
   gatgacaact ctccttcctt tatccaaatt cgctcagttg ccaagaagca tcctaaaact 1200
   tgggtacatt acattgctgc tgaagaggag gactgggact atgctccctt agtcctcgcc 1260
   cccgatgaca gaagttataa aagtcaatat ttgaacaatg gccctcagcg gattggtagg 1320
   aagtacaaaa aagtccgatt tatggcatac acagatgaaa cctttaagac tcgtgaagct 1380
   attcagcatg aatcaggaat cttgggacct ttactttatg gggaagttgg agacacactg 1440
   ttgattatat ttaagaatca agcaagcaga ccatataaca tctaccctca cggaatcact 1500
   gatgtccgtc ctttgtattc aaggagatta ccaaaaggtg taaaacattt gaaggatttt 1560
   ccaattctgc caggagaaat attcaaatat aaatggacag tgactgtaga agatgggcca 1620
   actaaatcag atcctcggtg cctgacccgc tattactcta gtttcgttaa tatggagaga 1680
   gatctagctt caggactcat tggccctctc ctcatctgct acaaagaatc tgtagatcaa 1740
   agaggaaacc agataatgtc agacaagagg aatgtcatcc tgttttctgt atttgatgag 1800
   aaccgaagct ggtacctcac agagaatata caacgctttc tccccaatcc agctggagtg 1860
   cagcttgagg atccagagtt ccaagcctcc aacatcatgc acagcatcaa tggctatgtt 1920
   tttgatagtt tgcagttgtc agtttgtttg catgaggtgg catactggta cattctaagc 1980
   attggagcac agactgactt cctttctgtc ttcttctctg gatatacctt caaacacaaa 2040
   atggtctatg aagacacact caccctattc ccattctcag gagaaactgt cttcatgtcg 2100
   atggaaaacc caggtctatg gattctgggg tgccacaact cagactttcg gaacagaggc 2160
   atgaccgcct tactgaaggt ttctagttgt gacaagaaca ctggtgatta ttacgaggac 2220
   agttatgaag atatttcagc atacttgctg agtaaaaaca atgccattga accaagaagc 2280
   ttctcccagc aaaacccacc agtcttgaaa cgccatcaac gggaaataac tcgtactact 2340
   cttcagtcag atcaagagga aattgactat gatgatacca tatcagttga aatgaagaag 2400
   gaagattttg acatttatga tgaggatgaa aatcagagcc cccgcagctt tcaaaagaaa 2460
   acacgacact attttattgc tgcagtggag aggctctggg attatgggat gagtagctcc 2520
   ccacatgttc taagaaacag ggctcagagt ggcagtgtcc ctcagttcaa gaaagttgtt 2580
   ttccaggaat ttactgatgg ctcctttact cagcccttat accgtggaga actaaatgaa 2640
   catttgggac tcctggggcc atatataaga gcagaagttg aagataatat catggtaact 2700
   ttcagaaatc aggcctctcg tccctattcc ttctattcta gccttatttc ttatgaggaa 2760
   gatcagaggc aaggagcaga acctagaaaa aactttgtca agcctaatga aaccaaaact 2820
   tacttttgga aagtgcaaca tcatatggca cccactaaag atgagtttga ctgcaaagcc 2880
   tgggcttatt tctctgatgt tgacctggaa aaagatgtgc actcaggcct gattggaccc     2940
   cttctggtct gccacactaa cacactgaac cctgctcatg ggagacaagt gacagtacag     3000
   gaatttgctc tgtttttcac catctttgat gagaccaaaa gctggtactt cactgaaaat     3060
   atggaaagaa actgcagggc tccctgcaat atccagatgg aagatcccac ttttaaagag     3120
   aattatcgct tccatgcaat caatggctac ataatggata cactacctgg cttagtaatg     3180
   gctcaggatc aaaggattcg atggtatctg ctcagcatgg gcagcaatga aaacatccat     3240
   tctattcatt tcagtggaca tgtgttcact gtacgaaaaa aagaggagta taaaatggca     3300
   ctgtacaatc tctatccagg tgtttttgag acagtggaaa tgttaccatc caaagctgga     3360
   atttggcggg tggaatgcct tattggcgag catctacatg ctgggatgag cacacttttt     3420
   ctggtgtaca gcaataagtg tcagactccc ctgggaatgg cttctggaca cattagagat     3480
   tttcagatta cagcttcagg acaatatgga cagtgggccc caaagctggc cagacttcat     3540
   tattccggat caatcaatgc ctggagcacc aaggagccct tttcttggat caaggtggat     3600
   ctgttggcac caatgattat tcacggcatc aagacccagg gtgcccgtca gaagttctcc     3660
   agcctctaca tctctcagtt tatcatcatg tatagtcttg atgggaagaa gtggcagact     3720
   tatcgaggaa attccactgg aaccttaatg gtcttctttg gcaatgtgga ttcatctggg     3780
   ataaaacaca atatttttaa ccctccaatt attgctcgat acatccgttt gcacccaact     3840
   cattatagca ttcgcagcac tcttcgcatg gagttgatgg gctgtgattt aaatagttgc     3900
   agcatgccat tgggaatgga gagtaaagca atatcagatg cacagattac tgcttcatcc     3960
   tactttacca atatgtttgc cacctggtct ccttcaaaag ctcgacttca cctccaaggg     4020
   aggagtaatg cctggagacc tcaggtgaat aatccaaaag agtggctgca agtggacttc     4080
   cagaagacaa tgaaagtcac aggagtaact actcagggag taaaatctct gcttaccagc     4140
   atgtatgtga aggagttcct catctccagc agtcaagatg gccatcagtg gactctcttt     4200
   tttcagaatg gcaaagtaaa ggtttttcag ggaaatcaag actccttcac acctgtggtg     4260
   aactctctag acccaccgtt actgactcgc taccttcgaa ttcaccccca gagttgggtg     4320
   caccagattg ccctgaggat ggaggttctg ggctgcgagg cacaggacct ctactga        4377
<210> 10
   <211> 1458
   <212> PRT
   <213> Homo sapiens
<220>
   <221> PEPTIDE
   <222> (1)..(1458)
<400> 10
   Met Gln Ile Glu Leu Ser Thr Cys Phe Phe Leu Cys Leu Leu Arg Phe
   1               5                   10                  15
   Cys Phe Ser Ala Thr Arg Arg Tyr Tyr Leu Gly Ala Val Glu Leu Ser
               20                  25                  30
   Trp Asp Tyr Met Gln Ser Asp Leu Gly Glu Leu Pro Val Asp Ala Arg
           35                  40                  45
   Phe Pro Pro Arg Val Pro Lys Ser Phe Pro Phe Asn Thr Ser Val Val
       50                  55                  60
   Tyr Lys Lys Thr Leu Phe Val Glu Phe Thr Asp His Leu Phe Asn Ile
   65                  70                  75                  80
   Ala Lys Pro Arg Pro Pro Trp Met Gly Leu Leu Gly Pro Thr Ile Gln
                   85                  90                  95
   Ala Glu Val Tyr Asp Thr Val Val Ile Thr Leu Lys Asn Met Ala Ser
               100                 105                 110
   His Pro Val Ser Leu His Ala Val Gly Val Ser Tyr Trp Lys Ala Ser
           115                 120                 125
   Glu Gly Ala Glu Tyr Asp Asp Gln Thr Ser Gln Arg Glu Lys Glu Asp
       130                 135                 140
   Asp Lys Val Phe Pro Gly Gly Ser His Thr Tyr Val Trp Gln Val Leu
   145                 150                 155                 160
   Lys Glu Asn Gly Pro Met Ala Ser Asp Pro Leu Cys Leu Thr Tyr Ser
                   165                 170                 175
   Tyr Leu Ser His Val Asp Leu Val Lys Asp Leu Asn Ser Gly Leu Ile
               180                 185                 190
   Gly Ala Leu Leu Val Cys Arg Glu Gly Ser Leu Ala Lys Glu Lys Thr
           195                 200                 205
   Gln Thr Leu His Lys Phe Ile Leu Leu Phe Ala Val Phe Asp Glu Gly
       210                 215                 220
   Lys Ser Trp His Ser Glu Thr Lys Asn Ser Leu Met Gln Asp Arg Asp
   225                 230                 235                 240
   Ala Ala Ser Ala Arg Ala Trp Pro Lys Met His Thr Val Asn Gly Tyr
                   245                 250                 255
   Val Asn Arg Ser Leu Pro Gly Leu Ile Gly Cys His Arg Lys Ser Val
   260 265 270
   Tyr Trp His Val Ile Gly Met Gly Thr Thr Pro Glu Val His Ser Ile
   275 280 285
   Phe Leu Glu Gly His Thr Phe Leu Val Arg Asn His Arg Gln Ala Ser
   290 295 300
   Leu Glu Ile Ser Pro Ile Thr Phe Leu Thr Ala Gln Thr Leu Leu Met
   305 310 315 320
   Asp Leu Gly Gln Phe Leu Leu Ser Cys His Ile Ser Ser His Gln His
   325 330 335
   Asp Gly Met Glu Ala Tyr Val Lys Val Asp Ser Cys Pro Glu Glu Pro
   340 345 350
   Gln Leu Arg Met Lys Asn Asn Glu Glu Ala Glu Asp Tyr Asp Asp Asp
   355 360 365
   Leu Thr Asp Ser Glu Met Asp Val Val Arg Phe Asp Asp Asp Asn Ser
   370 375 380
   Pro Ser Phe Ile Gln Ile Arg Ser Val Ala Lys Lys His Pro Lys Thr
   385 390 395 400
   Trp Val His Tyr Ile Ala Ala Glu Glu Glu Asp Trp Asp Tyr Ala Pro
   405 410 415
   Leu Val Leu Ala Pro Asp Asp Arg Ser Tyr Lys Ser Gln Tyr Leu Asn
   420 425 430
   Asn Gly Pro Gln Arg Ile Gly Arg Lys Tyr Lys Lys Val Arg Phe Met
   435 440 445
   Ala Tyr Thr Asp Glu Thr Phe Lys Thr Arg Glu Ala Ile Gln His Glu
   450 455 460
   Ser Gly Ile Leu Gly Pro Leu Leu Tyr Gly Glu Val Gly Asp Thr Leu
   465 470 475 480
   Leu Ile Ile Phe Lys Asn Gln Ala Ser Arg Pro Tyr Asn Ile Tyr Pro
   485 490 495
   His Gly Ile Thr Asp Val Arg Pro Leu Tyr Ser Arg Arg Leu Pro Lys
               500                 505                 510
   Gly Val Lys His Leu Lys Asp Phe Pro Ile Leu Pro Gly Glu Ile Phe
           515                 520                 525
   Lys Tyr Lys Trp Thr Val Thr Val Glu Asp Gly Pro Thr Lys Ser Asp
       530                 535                 540
   Pro Arg Cys Leu Thr Arg Tyr Tyr Ser Ser Phe Val Asn Met Glu Arg
   545                 550                 555                 560
   Asp Leu Ala Ser Gly Leu Ile Gly Pro Leu Leu Ile Cys Tyr Lys Glu
                   565                 570                 575
   Ser Val Asp Gln Arg Gly Asn Gln Ile Met Ser Asp Lys Arg Asn Val
               580                 585                 590
   Ile Leu Phe Ser Val Phe Asp Glu Asn Arg Ser Trp Tyr Leu Thr Glu
           595                 600                 605
   Asn Ile Gln Arg Phe Leu Pro Asn Pro Ala Gly Val Gln Leu Glu Asp
       610                 615                 620
   Pro Glu Phe Gln Ala Ser Asn Ile Met His Ser Ile Asn Gly Tyr Val
   625                 630                 635                 640
   Phe Asp Ser Leu Gln Leu Ser Val Cys Leu His Glu Val Ala Tyr Trp
                   645                 650                 655
   Tyr Ile Leu Ser Ile Gly Ala Gln Thr Asp Phe Leu Ser Val Phe Phe
               660                 665                 670
   Ser Gly Tyr Thr Phe Lys His Lys Met Val Tyr Glu Asp Thr Leu Thr
           675                 680                 685
   Leu Phe Pro Phe Ser Gly Glu Thr Val Phe Met Ser Met Glu Asn Pro
       690                 695                 700
   Gly Leu Trp Ile Leu Gly Cys His Asn Ser Asp Phe Arg Asn Arg Gly
   705                 710                 715                 720
   Met Thr Ala Leu Leu Lys Val Ser Ser Cys Asp Lys Asn Thr Gly Asp
                   725                 730                 735
   Tyr Tyr Glu Asp Ser Tyr Glu Asp Ile Ser Ala Tyr Leu Leu Ser Lys
               740                 745                 750
   Asn Asn Ala Ile Glu Pro Arg Ser Phe Ser Gln Gln Asn Pro Pro Val
           755                 760                 765
   Leu Lys Arg His Gln Arg Glu Ile Thr Arg Thr Thr Leu Gln Ser Asp
       770                 775                 780
   Gln Glu Glu Ile Asp Tyr Asp Asp Thr Ile Ser Val Glu Met Lys Lys
   785                 790                 795                 800
   Glu Asp Phe Asp Ile Tyr Asp Glu Asp Glu Asn Gln Ser Pro Arg Ser
                   805                 810                 815
   Phe Gln Lys Lys Thr Arg His Tyr Phe Ile Ala Ala Val Glu Arg Leu
               820                 825                 830
   Trp Asp Tyr Gly Met Ser Ser Ser Pro His Val Leu Arg Asn Arg Ala
           835                 840                 845
   Gln Ser Gly Ser Val Pro Gln Phe Lys Lys Val Val Phe Gln Glu Phe
       850                 855                 860
   Thr Asp Gly Ser Phe Thr Gln Pro Leu Tyr Arg Gly Glu Leu Asn Glu
   865                 870                 875                 880
   His Leu Gly Leu Leu Gly Pro Tyr Ile Arg Ala Glu Val Glu Asp Asn
                   885                 890                 895
   Ile Met Val Thr Phe Arg Asn Gln Ala Ser Arg Pro Tyr Ser Phe Tyr
               900                 905                 910
   Ser Ser Leu Ile Ser Tyr Glu Glu Asp Gln Arg Gln Gly Ala Glu Pro
           915                 920                 925
   Arg Lys Asn Phe Val Lys Pro Asn Glu Thr Lys Thr Tyr Phe Trp Lys
       930                 935                 940
   Val Gln His His Met Ala Pro Thr Lys Asp Glu Phe Asp Cys Lys Ala
   945                 950                 955                 960
   Trp Ala Tyr Phe Ser Asp Val Asp Leu Glu Lys Asp Val His Ser Gly
                   965                 970                 975
   Leu Ile Gly Pro Leu Leu Val Cys His Thr Asn Thr Leu Asn Pro Ala
               980                 985                 990
   His Gly Arg Gln Val Thr Val Gln  Glu Phe Ala Leu Phe  Phe Thr Ile
           995                 1000                1005
   Phe Asp  Glu Thr Lys Ser Trp  Tyr Phe Thr Glu Asn  Met Glu Arg
       1010                 1015                 1020
   Asn Cys  Arg Ala Pro Cys Asn  Ile Gln Met Glu Asp  Pro Thr Phe
       1025                 1030                 1035
   Lys Glu  Asn Tyr Arg Phe His  Ala Ile Asn Gly Tyr  Ile Met Asp
       1040                 1045                 1050
   Thr Leu  Pro Gly Leu Val Met  Ala Gln Asp Gln Arg  Ile Arg Trp
       1055                 1060                 1065
   Tyr Leu  Leu Ser Met Gly Ser  Asn Glu Asn Ile His  Ser Ile His
       1070                 1075                 1080
   Phe Ser  Gly His Val Phe Thr  Val Arg Lys Lys Glu  Glu Tyr Lys
       1085                 1090                 1095
   Met Ala  Leu Tyr Asn Leu Tyr  Pro Gly Val Phe Glu  Thr Val Glu
       1100                 1105                 1110
   Met Leu  Pro Ser Lys Ala Gly  Ile Trp Arg Val Glu  Cys Leu Ile
       1115                 1120                 1125
   Gly Glu  His Leu His Ala Gly  Met Ser Thr Leu Phe  Leu Val Tyr
       1130                 1135                 1140
   Ser Asn  Lys Cys Gln Thr Pro  Leu Gly Met Ala Ser  Gly His Ile
       1145                 1150                 1155
   Arg Asp  Phe Gln Ile Thr Ala  Ser Gly Gln Tyr Gly  Gln Trp Ala
       1160                 1165                 1170
   Pro Lys  Leu Ala Arg Leu His  Tyr Ser Gly Ser Ile  Asn Ala Trp
       1175                 1180                 1185
   Ser Thr  Lys Glu Pro Phe Ser  Trp Ile Lys Val Asp  Leu Leu Ala
       1190                 1195                 1200
   Pro Met  Ile Ile His Gly Ile  Lys Thr Gln Gly Ala  Arg Gln Lys
       1205                 1210                 1215
   Phe Ser  Ser Leu Tyr Ile Ser  Gln Phe Ile Ile Met  Tyr Ser Leu
       1220                 1225                 1230
   Asp Gly  Lys Lys Trp Gln Thr  Tyr Arg Gly Asn Ser  Thr Gly Thr
       1235                 1240                 1245
   Leu Met Val Phe Phe Gly Asn Val Asp Ser Ser Gly Ile Lys His
       1250                1255                1260
   Asn Ile Phe Asn Pro Pro Ile Ile Ala Arg Tyr Ile Arg Leu His
       1265                1270                1275
   Pro Thr His Tyr Ser Ile Arg Ser Thr Leu Arg Met Glu Leu Met
       1280                1285                1290
   Gly Cys Asp Leu Asn Ser Cys Ser Met Pro Leu Gly Met Glu Ser
       1295                1300                1305
   Lys Ala Ile Ser Asp Ala Gln Ile Thr Ala Ser Ser Tyr Phe Thr
       1310                1315                1320
   Asn Met Phe Ala Thr Trp Ser Pro Ser Lys Ala Arg Leu His Leu
       1325                1330                1335
   Gln Gly Arg Ser Asn Ala Trp Arg Pro Gln Val Asn Asn Pro Lys
       1340                1345                1350
   Glu Trp Leu Gln Val Asp Phe Gln Lys Thr Met Lys Val Thr Gly
       1355                1360                1365
   Val Thr Thr Gln Gly Val Lys Ser Leu Leu Thr Ser Met Tyr Val
       1370                1375                1380
   Lys Glu Phe Leu Ile Ser Ser Ser Gln Asp Gly His Gln Trp Thr
       1385                1390                1395
   Leu Phe Phe Gln Asn Gly Lys Val Lys Val Phe Gln Gly Asn Gln
       1400                1405                1410
   Asp Ser Phe Thr Pro Val Val Asn Ser Leu Asp Pro Pro Leu Leu
       1415                1420                1425
   Thr Arg Tyr Leu Arg Ile His Pro Gln Ser Trp Val His Gln Ile
       1430                1435                1440
   Ala Leu Arg Met Glu Val Leu Gly Cys Glu Ala Gln Asp Leu Tyr
       1445                1450                1455
<210> 11
   <211> 4377
   <212> DNA
   <213> Homo sapiens
<220>
   <221> gene
   <222> (1)..(4377)
<400> 11
   atgcaaatag agctctccac ctgcttcttt ctgtgccttt tgcgattctg ctttagtgcc       60
   accagaagat actacctggg tgcagtggaa ctgtcatggg actatatgca aagtgatctc      120
   ggtgagctgc ctgtggacgc aagatttcct cctagagtgc caaaatcttt tccattcaac      180
   acctcagtcg tgtacaaaaa gactctgttt gtagaattca cggatcacct tttcaacatc      240
   gctaagccaa ggccaccctg gatgggtctg ctaggtccta ccatccaggc tgaggtttat      300
   gatacagtgg tcattacact taagaacatg gcttcccatc ctgtcagtct tcatgctgtt      360
   ggtgtatcct actggaaagc ttctgaggga gctgaatatg atgatcagac cagtcaaagg      420
   gagaaagaag atgataaagt cttccctggt ggaagccata catatgtctg gcaggtcctg      480
   aaagagaatg gtccaatggc ctctgaccca ctgtgcctta cctactcata tctttctcat      540
   gtggacctgg taaaagactt gaattcaggc ctcattggag ccctactagt atgtagagaa      600
   gggagtctgg ccaaggaaaa gacacagacc ttgcacaaat ttatactact ttttgctgta       660
   tttgatgaag ggaaaagttg gcactcagaa acaaagaact ccttgatgca ggatagggat      720
   gctgcatctg ctcgggcctg gcctaaaatg cacacagtca atggttatgt aaacaggtct      780
   ctgccaggtc tgattggatg ccacaggaaa tcagtctatt ggcatgtgat tggaatgggc      840
   accactcctg aagtgcactc aatattcctc gaaggtcaca catttcttgt gaggaaccat      900
   cgccaggcgt ccttggaaat ctcgccaata actttcctta ctgctcaaac actcttgatg      960
   gaccttggac agtttctact gttttgtcat atctcttccc accaacatga tggcatggaa     1020
   gcttatgtca aagtagacag ctgtccagag gaaccccaac tacgaatgaa aaataatgaa     1080
   gaagcggaag actatgatga tgatcttact gattctgaaa tggatgtggt caggtttgat     1140
   gatgacaact ctccttcctt tatccaaatt cgctcagttg ccaagaagca tcctaaaact     1200
   tgggtacatt acattgctgc tgaagaggag gactgggact atgctccctt agtcctcgcc     1260
   cccgatgaca gaagttataa aagtcaatat ttgaacaatg gccctcagcg gattggtagg     1320
   aagtacaaaa aagtccgatt tatggcatac acagatgaaa cctttaagac tcgtgaagct     1380
   attcagcatg aatcaggaat cttgggacct ttactttatg gggaagttgg agacacactg     1440
   ttgattatat ttaagaatca agcaagcaga ccatataaca tctaccctca cggaatcact     1500
   gatgtccgtc ctttgtattc aaggagatta ccaaaaggtg taaaacattt gaaggatttt     1560
   ccaattctgc caggagaaat attcaaatat aaatggacag tgactgtaga agttgggcca     1620
   actaaatcag atcctcggtg cctgacccgc tattactcta gtttcgttaa tatggagaga     1680
   gatctagctt caggactcat tggccctctc ctcatctgct acaaagaatc tgtagatcaa     1740
   agaggaaacc agataatgtc agacaagagg aatgtcatcc tgttttctgt atttgatgag 1800
   aaccgaagct ggtacctcac agagaatata caacgctttc tccccaatcc agctggagtg 1860
   cagcttgagg atccagagtt ccaagcctcc aacatcatgc acagcatcaa tggctatgtt 1920
   tttgatagtt tgcagttgtc agtttgtttg catgaggtgg catactggta cattctaagc 1980
   attggagcac agactgactt cctttctgtc ttcttctctg gatatacctt caaacacaaa 2040
   atggtctatg aagacacact caccctattc ccattctcag gagaaactgt cttcatgtcg 2100
   atggaaaacc caggtctatg gattctgggg tgccacaact cagactttcg gaacagaggc 2160
   atgaccgcct tactgaaggt ttctagttgt gacaagaaca ctggtgatta ttacgaggac 2220
   agttatgaag atatttcagc atacttgctg agtaaaaaca atgccattga accaagaagc 2280
   ttctcccagc aaaacccacc agtcttgaaa cgccatcaac gggaaataac tcgtactact 2340
   cttcagtcag atcaagagga aattgactat gatgatacca tatcagttga aatgaagaag 2400
   gaagattttg acatttatga tgaggatgaa aatcagagcc cccgcagctt tcaaaagaaa 2460
   acacgacact attttattgc tgcagtggag aggctctggg attatgggat gagtagctcc 2520
   ccacatgttc taagaaacag ggctcagagt ggcagtgtcc ctcagttcaa gaaagttgtt 2580
   ttccaggaat ttactgatgg ctcctttact cagcccttat accgtggaga actaaatgaa 2640
   catttgggac tcctggggcc atatataaga gcagaagttg aagataatat catggtaact 2700
   ttcagaaatc aggcctctcg tccctattcc ttctattcta gccttatttc ttatgaggaa 2760
   gatcagaggc aaggagcaga acctagaaaa aactttgtca agcctaatga aaccaaaact 2820
   tacttttgga aagtgcaaca tcatatggca cccactaaag atgagtttga ctgcaaagcc 2880
   tgggcttatt tctctgatgt tgacctggaa aaagatgtgc actcaggcct gattggaccc 2940
   cttctggtct gccacactaa cacactgaac cctgctcatg ggagacaagt gacagtacag 3000
   gaatttgctc tgtttttcac catctttgat gagaccaaaa gctggtactt cactgaaaat 3060
   atggaaagaa actgcagggc tccctgcaat atccagatgg aagatcccac ttttaaagag 3120
   aattatcgct tccatgcaat caatggctac ataatggata cactacctgg cttagtaatg 3180
   gctcaggatc aaaggattcg atggtatctg ctcagcatgg gcagcaatga aaacatccat 3240
   tctattcatt tcagtggaca tgtgttcact gtacgaaaaa aagaggagta taaaatggca 3300
   ctgtacaatc tctatccagg tgtttttgag acagtggaaa tgttaccatc caaagctgga 3360
   atttggcggg tggaatgcct tattggcgag catctacatg ctgggatgag cacacttttt 3420
   ctggtgtaca gcaataagtg tcagactccc ctgggaatgg cttctggaca cattagagat 3480
   tttcagatta cagcttcagg acaatatgga cagtgggccc caaagctggc cagacttcat 3540
   tattccggat caatcaatgc ctggagcacc aaggagccct tttcttggat caaggtggat 3600
   ctgttggcac caatgattat tcacggcatc aagacccagg gtgcccgtca gaagttctcc      3660
   agcctctaca tctctcagtt tatcatcatg tatagtcttg atgggaagaa gtggcagact      3720
   tatcgaggaa attccactgg aaccttaatg gtcttctttg gcaatgtgga ttcatctggg      3780
   ataaaacaca atatttttaa ccctccaatt attgctcgat acatccgttt gcacccaact      3840
   cattatagca ttcgcagcac tcttcgcatg gagttgatgg gctgtgattt aaatagttgc      3900
   agcatgccat tgggaatgga gagtaaagca atatcagatg cacagattac tgcttcatcc      3960
   tactttacca atatgtttgc cacctggtct ccttcaaaag ctcgacttca cctccaaggg      4020
   aggagtaatg cctggagacc tcaggtgaat aatccaaaag agtggctgca agtggacttc      4080
   cagaagacaa tgaaagtcac aggagtaact actcagggag taaaatctct gcttaccagc      4140
   atgtatgtga aggagttcct catctccagc agtcaagatg gccatcagtg gactctcttt      4200
   tttcagaatg gcaaagtaaa ggtttttcag ggaaatcaag actccttcac acctgtggtg      4260
   aactctctag acccaccgtt actgactcgc taccttcgaa ttcaccccca gagttgggtg      4320
   caccagattg ccctgaggat ggaggttctg ggctgcgagg cacaggacct ctactga         4377
<210> 12
   <211> 1458
   <212> PRT
   <213> Homo sapiens
<220>
   <221> PEPTIDE
   <222> (1)..(1458)
<400> 12
   Met Gln Ile Glu Leu Ser Thr Cys Phe Phe Leu Cys Leu Leu Arg Phe
   1               5                   10                  15
   Cys Phe Ser Ala Thr Arg Arg Tyr Tyr Leu Gly Ala Val Glu Leu Ser
               20                  25                  30
   Trp Asp Tyr Met Gln Ser Asp Leu Gly Glu Leu Pro Val Asp Ala Arg
           35                  40                  45
   Phe Pro Pro Arg Val Pro Lys Ser Phe Pro Phe Asn Thr Ser Val Val
       50                  55                  60
   Tyr Lys Lys Thr Leu Phe Val Glu Phe Thr Asp His Leu Phe Asn Ile
   65                  70                  75                  80
   Ala Lys Pro Arg Pro Pro Trp Met Gly Leu Leu Gly Pro Thr Ile Gln
                   85                  90                  95
   Ala Glu Val Tyr Asp Thr Val Val Ile Thr Leu Lys Asn Met Ala Ser
               100                 105                 110
   His Pro Val Ser Leu His Ala Val Gly Val Ser Tyr Trp Lys Ala Ser
           115                 120                 125
   Glu Gly Ala Glu Tyr Asp Asp Gln Thr Ser Gln Arg Glu Lys Glu Asp
       130                 135                 140
   Asp Lys Val Phe Pro Gly Gly Ser His Thr Tyr Val Trp Gln Val Leu
   145                 150                 155                 160
   Lys Glu Asn Gly Pro Met Ala Ser Asp Pro Leu Cys Leu Thr Tyr Ser
                   165                 170                 175
   Tyr Leu Ser His Val Asp Leu Val Lys Asp Leu Asn Ser Gly Leu Ile
               180                 185                 190
   Gly Ala Leu Leu Val Cys Arg Glu Gly Ser Leu Ala Lys Glu Lys Thr
           195                 200                 205
   Gln Thr Leu His Lys Phe Ile Leu Leu Phe Ala Val Phe Asp Glu Gly
       210                 215                 220
   Lys Ser Trp His Ser Glu Thr Lys Asn Ser Leu Met Gln Asp Arg Asp
   225                 230                 235                 240
   Ala Ala Ser Ala Arg Ala Trp Pro Lys Met His Thr Val Asn Gly Tyr
                   245                 250                 255
   Val Asn Arg Ser Leu Pro Gly Leu Ile Gly Cys His Arg Lys Ser Val
               260                 265                 270
   Tyr Trp His Val Ile Gly Met Gly Thr Thr Pro Glu Val His Ser Ile
           275                 280                 285
   Phe Leu Glu Gly His Thr Phe Leu Val Arg Asn His Arg Gln Ala Ser
       290                 295                 300
   Leu Glu Ile Ser Pro Ile Thr Phe Leu Thr Ala Gln Thr Leu Leu Met
   305                 310                 315                 320
   Asp Leu Gly Gln Phe Leu Leu Phe Cys His Ile Ser Ser His Gln His
                   325                 330                 335
   Asp Gly Met Glu Ala Tyr Val Lys Val Asp Ser Cys Pro Glu Glu Pro
               340                 345                 350
   Gln Leu Arg Met Lys Asn Asn Glu Glu Ala Glu Asp Tyr Asp Asp Asp
           355                 360                 365
   Leu Thr Asp Ser Glu Met Asp Val Val Arg Phe Asp Asp Asp Asn Ser
       370                 375                 380
   Pro Ser Phe Ile Gln Ile Arg Ser Val Ala Lys Lys His Pro Lys Thr
   385                 390                 395                 400
   Trp Val His Tyr Ile Ala Ala Glu Glu Glu Asp Trp Asp Tyr Ala Pro
                   405                 410                 415
   Leu Val Leu Ala Pro Asp Asp Arg Ser Tyr Lys Ser Gln Tyr Leu Asn
               420                 425                 430
   Asn Gly Pro Gln Arg Ile Gly Arg Lys Tyr Lys Lys Val Arg Phe Met
           435                 440                 445
   Ala Tyr Thr Asp Glu Thr Phe Lys Thr Arg Glu Ala Ile Gln His Glu
       450                 455                 460
   Ser Gly Ile Leu Gly Pro Leu Leu Tyr Gly Glu Val Gly Asp Thr Leu
   465                 470                 475                 480
   Leu Ile Ile Phe Lys Asn Gln Ala Ser Arg Pro Tyr Asn Ile Tyr Pro
                   485                 490                 495
   His Gly Ile Thr Asp Val Arg Pro Leu Tyr Ser Arg Arg Leu Pro Lys
               500                 505                 510
   Gly Val Lys His Leu Lys Asp Phe Pro Ile Leu Pro Gly Glu Ile Phe
           515                 520                 525
   Lys Tyr Lys Trp Thr Val Thr Val Glu Val Gly Pro Thr Lys Ser Asp
       530                 535                 540
   Pro Arg Cys Leu Thr Arg Tyr Tyr Ser Ser Phe Val Asn Met Glu Arg
   545                 550                 555                 560
   Asp Leu Ala Ser Gly Leu Ile Gly Pro Leu Leu Ile Cys Tyr Lys Glu
                   565                 570                 575
   Ser Val Asp Gln Arg Gly Asn Gln Ile Met Ser Asp Lys Arg Asn Val
               580                 585                 590
   Ile Leu Phe Ser Val Phe Asp Glu Asn Arg Ser Trp Tyr Leu Thr Glu
           595                 600                 605
   Asn Ile Gln Arg Phe Leu Pro Asn Pro Ala Gly Val Gln Leu Glu Asp
       610                 615                 620
   Pro Glu Phe Gln Ala Ser Asn Ile Met His Ser Ile Asn Gly Tyr Val
   625                 630                 635                 640
   Phe Asp Ser Leu Gln Leu Ser Val Cys Leu His Glu Val Ala Tyr Trp
                   645                 650                 655
   Tyr Ile Leu Ser Ile Gly Ala Gln Thr Asp Phe Leu Ser Val Phe Phe
               660                 665                 670
   Ser Gly Tyr Thr Phe Lys His Lys Met Val Tyr Glu Asp Thr Leu Thr
           675                 680                 685
   Leu Phe Pro Phe Ser Gly Glu Thr Val Phe Met Ser Met Glu Asn Pro
       690                 695                 700
   Gly Leu Trp Ile Leu Gly Cys His Asn Ser Asp Phe Arg Asn Arg Gly
   705                 710                 715                 720
   Met Thr Ala Leu Leu Lys Val Ser Ser Cys Asp Lys Asn Thr Gly Asp
                   725                 730                 735
   Tyr Tyr Glu Asp Ser Tyr Glu Asp Ile Ser Ala Tyr Leu Leu Ser Lys
               740                 745                 750
   Asn Asn Ala Ile Glu Pro Arg Ser Phe Ser Gln Gln Asn Pro Pro Val
           755                 760                 765
   Leu Lys Arg His Gln Arg Glu Ile Thr Arg Thr Thr Leu Gln Ser Asp
       770                 775                 780
   Gln Glu Glu Ile Asp Tyr Asp Asp Thr Ile Ser Val Glu Met Lys Lys
   785                 790                 795                 800
   Glu Asp Phe Asp Ile Tyr Asp Glu Asp Glu Asn Gln Ser Pro Arg Ser
                   805                 810                 815
   Phe Gln Lys Lys Thr Arg His Tyr Phe Ile Ala Ala Val Glu Arg Leu
               820                 825                 830
   Trp Asp Tyr Gly Met Ser Ser Ser Pro His Val Leu Arg Asn Arg Ala
           835                 840                 845
   Gln Ser Gly Ser Val Pro Gln Phe Lys Lys Val Val Phe Gln Glu Phe
   850 855 860
   Thr Asp Gly Ser Phe Thr Gln Pro Leu Tyr Arg Gly Glu Leu Asn Glu
   865 870 875 880
   His Leu Gly Leu Leu Gly Pro Tyr Ile Arg Ala Glu Val Glu Asp Asn
   885 890 895
   Ile Met Val Thr Phe Arg Asn Gln Ala Ser Arg Pro Tyr Ser Phe Tyr
   900 905 910
   Ser Ser Leu Ile Ser Tyr Glu Glu Asp Gln Arg Gln Gly Ala Glu Pro
   915 920 925
   Arg Lys Asn Phe Val Lys Pro Asn Glu Thr Lys Thr Tyr Phe Trp Lys
   930 935 940
   Val Gln His His Met Ala Pro Thr Lys Asp Glu Phe Asp Cys Lys Ala
   945 950 955 960
   Trp Ala Tyr Phe Ser Asp Val Asp Leu Glu Lys Asp Val His Ser Gly
   965 970 975
   Leu Ile Gly Pro Leu Leu Val Cys His Thr Asn Thr Leu Asn Pro Ala
   980 985 990
   His Gly Arg Gln Val Thr Val Gln Glu Phe Ala Leu Phe Phe Thr Ile
   995 1000 1005
   Phe Asp Glu Thr Lys Ser Trp Tyr Phe Thr Glu Asn Met Glu Arg
   1010 1015 1020
   Asn Cys Arg Ala Pro Cys Asn Ile Gln Met Glu Asp Pro Thr Phe
   1025 1030 1035
   Lys Glu Asn Tyr Arg Phe His Ala Ile Asn Gly Tyr Ile Met Asp
   1040 1045 1050
   Thr Leu Pro Gly Leu Val Met Ala Gln Asp Gln Arg Ile Arg Trp
   1055 1060 1065
   Tyr Leu Leu Ser Met Gly Ser Asn Glu Asn Ile His Ser Ile His
   1070 1075 1080
   Phe Ser Gly His Val Phe Thr Val Arg Lys Lys Glu Glu Tyr Lys
   1085 1090 1095
   Met Ala Leu Tyr Asn Leu Tyr Pro Gly Val Phe Glu Thr Val Glu
   1100 1105 1110
   Met Leu Pro Ser Lys Ala Gly Ile Trp Arg Val Glu Cys Leu Ile
   1115 1120 1125
   Gly Glu His Leu His Ala Gly Met Ser Thr Leu Phe Leu Val Tyr
   1130 1135 1140
   Ser Asn Lys Cys Gln Thr Pro Leu Gly Met Ala Ser Gly His Ile
   1145 1150 1155
   Arg Asp Phe Gln Ile Thr Ala Ser Gly Gln Tyr Gly Gln Trp Ala
   1160 1165 1170
   Pro Lys Leu Ala Arg Leu His Tyr Ser Gly Ser Ile Asn Ala Trp
   1175 1180 1185
   Ser Thr Lys Glu Pro Phe Ser Trp Ile Lys Val Asp Leu Leu Ala
   1190 1195 1200
   Pro Met Ile Ile His Gly Ile Lys Thr Gln Gly Ala Arg Gln Lys
   1205 1210 1215
   Phe Ser Ser Leu Tyr Ile Ser Gln Phe Ile Ile Met Tyr Ser Leu
   1220 1225 1230
   Asp Gly Lys Lys Trp Gln Thr Tyr Arg Gly Asn Ser Thr Gly Thr
   1235 1240 1245
   Leu Met Val Phe Phe Gly Asn Val Asp Ser Ser Gly Ile Lys His
   1250 1255 1260
   Asn Ile Phe Asn Pro Pro Ile Ile Ala Arg Tyr Ile Arg Leu His
   1265 1270 1275
   Pro Thr His Tyr Ser Ile Arg Ser Thr Leu Arg Met Glu Leu Met
   1280 1285 1290
   Gly Cys Asp Leu Asn Ser Cys Ser Met Pro Leu Gly Met Glu Ser
   1295 1300 1305
   Lys Ala Ile Ser Asp Ala Gln Ile Thr Ala Ser Ser Tyr Phe Thr
   1310 1315 1320
   Asn Met Phe Ala Thr Trp Ser Pro Ser Lys Ala Arg Leu His Leu
   1325 1330 1335
   Gln Gly  Arg Ser Asn Ala Trp  Arg Pro Gln Val Asn  Asn Pro Lys
       1340                 1345                 1350
   Glu Trp  Leu Gln Val Asp Phe  Gln Lys Thr Met Lys  Val Thr Gly
       1355                 1360                 1365
   Val Thr  Thr Gln Gly Val Lys  Ser Leu Leu Thr Ser  Met Tyr Val
       1370                 1375                 1380
   Lys Glu  Phe Leu Ile Ser Ser  Ser Gln Asp Gly His  Gln Trp Thr
       1385                 1390                 1395
   Leu Phe  Phe Gln Asn Gly Lys  Val Lys Val Phe Gln  Gly Asn Gln
       1400                 1405                 1410
   Asp Ser  Phe Thr Pro Val Val  Asn Ser Leu Asp Pro  Pro Leu Leu
       1415                 1420                 1425
   Thr Arg  Tyr Leu Arg Ile His  Pro Gln Ser Trp Val  His Gln Ile
       1430                 1435                 1440
   Ala Leu  Arg Met Glu Val Leu  Gly Cys Glu Ala Gln  Asp Leu Tyr
       1445                 1450                 1455
<210> 13
   <211> 4320
   <212> DNA
   <213> Homo sapiens
<220>
   <221> gene
   <222> (1)..(4320)
<400> 13
   gccaccagaa gatactacct gggtgcagtg gaactgtcat gggactatat gcaaagtgat        60
   ctcggtgagc tgcctgtgga cgcaagattt cctcctagag tgccaaaatc ttttccattc       120
   aacacctcag tcgtgtacaa aaagactctg tttgtagaat tcacggatca ccttttcaac       180
   atcgctaagc caaggccacc ctggatgggt ctgctaggtc ctaccatcca ggctgaggtt       240
   tatgatacag tggtcattac acttaagaac atggcttccc atcctgtcag tcttcatgct       300
   gttggtgtat cctactggaa agcttctgag ggagctgaat atgatgatca gaccagtcaa       360
   agggagaaag aagatgataa agtcttccct ggtggaagcc atacatatgt ctggcaggtc       420
   ctgaaagaga atggtccaat ggcctctgac ccactgtgcc ttacctactc atatctttct       480
   catgtggacc tggtaaaaga cttgaattca ggcctcattg gagccctact agtatgtaga       540
   gaagggagtc tggccaagga aaagacacag accttgcaca aatttatact actttttgct       600
   gtatttgatg aagggaaaag ttggcactca gaaacaaaga actccttgat gcaggatagg 660
   gatgctgcat ctgctcgggc ctggcctaaa atgcacacag tcaatggtta tgtaaacagg 720
   tctctgccag gtctgattgg atgccacagg aaatcagtct attggcatgt gattggaatg 780
   ggcaccactc ctgaagtgca ctcaatattc ctcgaaggtc acacatttct tgtgaggaac 840
   catcgccagg cgtccttgga aatctcgcca ataactttcc ttactgctca aacactcttg 900
   atggaccttg gacagtttct actgagttgt catatctctt cccaccaaca tgatggcatg 960
   gaagcttatg tcaaagtaga cagctgtcca gaggaacccc aactacgaat gaaaaataat 1020
   gaagaagcgg aagactatga tgatgatctt actgattctg aaatggatgt ggtcaggttt 1080
   gatgatgaca actctccttc ctttatccaa attcgctcag ttgccaagaa gcatcctaaa 1140
   acttgggtac attacattgc tgctgaagag gaggactggg actatgctcc cttagtcctc 1200
   gcccccgatg acagaagtta taaaagtcaa tatttgaaca atggccctca gcggattggt 1260
   aggaagtaca aaaaagtccg atttatggca tacacagatg aaacctttaa gactcgtgaa 1320
   gctattcagc atgaatcagg aatcttggga cctttacttt atggggaagt tggagacaca 1380
   ctgttgatta tatttaagaa tcaagcaagc agaccatata acatctaccc tcacggaatc 1440
   actgatgtcc gtcctttgta ttcaaggaga ttaccaaaag gtgtaaaaca tttgaaggat 1500
   tttccaattc tgccaggaga aatattcaaa tataaatgga cagtgactgt agaagttggg 1560
   ccaactaaat cagatcctcg gtgcctgacc cgctattact ctagtttcgt taatatggag 1620
   agagatctag cttcaggact cattggccct ctcctcatct gctacaaaga atctgtagat 1680
   caaagaggaa accagataat gtcagacaag aggaatgtca tcctgttttc tgtatttgat 1740
   gagaaccgaa gctggtacct cacagagaat atacaacgct ttctccccaa tccagctgga 1800
   gtgcagcttg aggatccaga gttccaagcc tccaacatca tgcacagcat caatggctat 1860
   gtttttgata gtttgcagtt gtcagtttgt ttgcatgagg tggcatactg gtacattcta 1920
   agcattggag cacagactga cttcctttct gtcttcttct ctggatatac cttcaaacac 1980
   aaaatggtct atgaagacac actcacccta ttcccattct caggagaaac tgtcttcatg 2040
   tcgatggaaa acccaggtct atggattctg gggtgccaca actcagactt tcggaacaga 2100
   ggcatgaccg ccttactgaa ggtttctagt tgtgacaaga acactggtga ttattacgag 2160
   gacagttatg aagatatttc agcatacttg ctgagtaaaa acaatgccat tgaaccaaga 2220
   agcttctccc agcaaaaccc accagtcttg aaacgccatc aacgggaaat aactcgtact 2280
   actcttcagt cagatcaaga ggaaattgac tatgatgata ccatatcagt tgaaatgaag 2340
   aaggaagatt ttgacattta tgatgaggat gaaaatcaga gcccccgcag ctttcaaaag 2400
   aaaacacgac actattttat tgctgcagtg gagaggctct gggattatgg gatgagtagc 2460
   tccccacatg ttctaagaaa cagggctcag agtggcagtg tccctcagtt caagaaagtt 2520
   gttttccagg aatttactga tggctccttt actcagccct tataccgtgg agaactaaat 2580
   gaacatttgg gactcctggg gccatatata agagcagaag ttgaagataa tatcatggta 2640
   actttcagaa atcaggcctc tcgtccctat tccttctatt ctagccttat ttcttatgag 2700
   gaagatcaga ggcaaggagc agaacctaga aaaaactttg tcaagcctaa tgaaaccaaa 2760
   acttactttt ggaaagtgca acatcatatg gcacccacta aagatgagtt tgactgcaaa 2820
   gcctgggctt atttctctga tgttgacctg gaaaaagatg tgcactcagg cctgattgga 2880
   ccccttctgg tctgccacac taacacactg aaccctgctc atgggagaca agtgacagta 2940
   caggaatttg ctctgttttt caccatcttt gatgagacca aaagctggta cttcactgaa 3000
   aatatggaaa gaaactgcag ggctccctgc aatatccaga tggaagatcc cacttttaaa 3060
   gagaattatc gcttccatgc aatcaatggc tacataatgg atacactacc tggcttagta 3120
   atggctcagg atcaaaggat tcgatggtat ctgctcagca tgggcagcaa tgaaaacatc 3180
   cattctattc atttcagtgg acatgtgttc actgtacgaa aaaaagagga gtataaaatg 3240
   gcactgtaca atctctatcc aggtgttttt gagacagtgg aaatgttacc atccaaagct 3300
   ggaatttggc gggtggaatg ccttattggc gagcatctac atgctgggat gagcacactt 3360
   tttctggtgt acagcaataa gtgtcagact cccctgggaa tggcttctgg acacattaga 3420
   gattttcaga ttacagcttc aggacaatat ggacagtggg ccccaaagct ggccagactt 3480
   cattattccg gatcaatcaa tgcctggagc accaaggagc ccttttcttg gatcaaggtg 3540
   gatctgttgg caccaatgat tattcacggc atcaagaccc agggtgcccg tcagaagttc 3600
   tccagcctct acatctctca gtttatcatc atgtatagtc ttgatgggaa gaagtggcag 3660
   acttatcgag gaaattccac tggaacctta atggtcttct ttggcaatgt ggattcatct 3720
   gggataaaac acaatatttt taaccctcca attattgctc gatacatccg tttgcaccca 3780
   actcattata gcattcgcag cactcttcgc atggagttga tgggctgtga tttaaatagt 3840
   tgcagcatgc cattgggaat ggagagtaaa gcaatatcag atgcacagat tactgcttca 3900
   tcctacttta ccaatatgtt tgccacctgg tctccttcaa aagctcgact tcacctccaa 3960
   gggaggagta atgcctggag acctcaggtg aataatccaa aagagtggct gcaagtggac 4020
   ttccagaaga caatgaaagt cacaggagta actactcagg gagtaaaatc tctgcttacc 4080
   agcatgtatg tgaaggagtt cctcatctcc agcagtcaag atggccatca gtggactctc 4140
   ttttttcaga atggcaaagt aaaggttttt cagggaaatc aagactcctt cacacctgtg 4200
   gtgaactctc tagacccacc gttactgact cgctaccttc gaattcaccc ccagagttgg 4260
   gtgcaccaga ttgccctgag gatggaggtt ctgggctgcg aggcacagga cctctactga 4320
<210> 14
   <211> 1439
   <212> PRT
   <213> Homo sapiens
<220>
   <221> PEPTIDE
   <222> (1)..(1439)
<400> 14
   Met Gln Ile Glu Leu Ser Thr Cys Phe Phe Leu Cys Leu Leu Arg Phe
   1               5                   10                  15
   Cys Phe Ser Ala Thr Arg Arg Tyr Tyr Leu Gly Ala Val Glu Leu Ser
               20                  25                  30
   Trp Asp Tyr Met Gln Ser Asp Leu Gly Glu Leu Pro Val Asp Ala Arg
           35                  40                  45
   Phe Pro Pro Arg Val Pro Lys Ser Phe Pro Phe Asn Thr Ser Val Val
       50                  55                  60
   Tyr Lys Lys Thr Leu Phe Val Glu Phe Thr Asp His Leu Phe Asn Ile
   65                  70                  75                  80
   Ala Lys Pro Arg Pro Pro Trp Met Gly Leu Leu Gly Pro Thr Ile Gln
                   85                  90                  95
   Ala Glu Val Tyr Asp Thr Val Val Ile Thr Leu Lys Asn Met Ala Ser
               100                 105                 110
   His Pro Val Ser Leu His Ala Val Gly Val Ser Tyr Trp Lys Ala Ser
           115                 120                 125
   Glu Gly Ala Glu Tyr Asp Asp Gln Thr Ser Gln Arg Glu Lys Glu Asp
       130                 135                 140
   Asp Lys Val Phe Pro Gly Gly Ser His Thr Tyr Val Trp Gln Val Leu
   145                 150                 155                 160
   Lys Glu Asn Gly Pro Met Ala Ser Asp Pro Leu Cys Leu Thr Tyr Ser
                   165                 170                 175
   Tyr Leu Ser His Val Asp Leu Val Lys Asp Leu Asn Ser Gly Leu Ile
               180                 185                 190
   Gly Ala Leu Leu Val Cys Arg Glu Gly Ser Leu Ala Lys Glu Lys Thr
           195                 200                 205
   Gln Thr Leu His Lys Phe Ile Leu Leu Phe Ala Val Phe Asp Glu Gly
       210                 215                 220
   Lys Ser Trp His Ser Glu Thr Lys Asn Ser Leu Met Gln Asp Arg Asp
   225                 230                 235                 240
   Ala Ala Ser Ala Arg Ala Trp Pro Lys Met His Thr Val Asn Gly Tyr
                   245                 250                 255
   Val Asn Arg Ser Leu Pro Gly Leu Ile Gly Cys His Arg Lys Ser Val
               260                 265                 270
   Tyr Trp His Val Ile Gly Met Gly Thr Thr Pro Glu Val His Ser Ile
           275                 280                 285
   Phe Leu Glu Gly His Thr Phe Leu Val Arg Asn His Arg Gln Ala Ser
       290                 295                 300
   Leu Glu Ile Ser Pro Ile Thr Phe Leu Thr Ala Gln Thr Leu Leu Met
   305                 310                 315                 320
   Asp Leu Gly Gln Phe Leu Leu Ser Cys His Ile Ser Ser His Gln His
                   325                 330                 335
   Asp Gly Met Glu Ala Tyr Val Lys Val Asp Ser Cys Pro Glu Glu Pro
               340                 345                 350
   Gln Leu Arg Met Lys Asn Asn Glu Glu Ala Glu Asp Tyr Asp Asp Asp
           355                 360                 365
   Leu Thr Asp Ser Glu Met Asp Val Val Arg Phe Asp Asp Asp Asn Ser
       370                 375                 380
   Pro Ser Phe Ile Gln Ile Arg Ser Val Ala Lys Lys His Pro Lys Thr
   385                 390                 395                 400
   Trp Val His Tyr Ile Ala Ala Glu Glu Glu Asp Trp Asp Tyr Ala Pro
                   405                 410                 415
   Leu Val Leu Ala Pro Asp Asp Arg Ser Tyr Lys Ser Gln Tyr Leu Asn
               420                 425                 430
   Asn Gly Pro Gln Arg Ile Gly Arg Lys Tyr Lys Lys Val Arg Phe Met
           435                 440                 445
   Ala Tyr Thr Asp Glu Thr Phe Lys Thr Arg Glu Ala Ile Gln His Glu
       450                 455                 460
   Ser Gly Ile Leu Gly Pro Leu Leu Tyr Gly Glu Val Gly Asp Thr Leu
   465 470 475 480
   Leu Ile Ile Phe Lys Asn Gln Ala Ser Arg Pro Tyr Asn Ile Tyr Pro
   485 490 495
   His Gly Ile Thr Asp Val Arg Pro Leu Tyr Ser Arg Arg Leu Pro Lys
   500 505 510
   Gly Val Lys His Leu Lys Asp Phe Pro Ile Leu Pro Gly Glu Ile Phe
   515 520 525
   Lys Tyr Lys Trp Thr Val Thr Val Glu Val Gly Pro Thr Lys Ser Asp
   530 535 540
   Pro Arg Cys Leu Thr Arg Tyr Tyr Ser Ser Phe Val Asn Met Glu Arg
   545 550 555 560
   Asp Leu Ala Ser Gly Leu Ile Gly Pro Leu Leu Ile Cys Tyr Lys Glu
   565 570 575
   Ser Val Asp Gln Arg Gly Asn Gln Ile Met Ser Asp Lys Arg Asn Val
   580 585 590
   Ile Leu Phe Ser Val Phe Asp Glu Asn Arg Ser Trp Tyr Leu Thr Glu
   595 600 605
   Asn Ile Gln Arg Phe Leu Pro Asn Pro Ala Gly Val Gln Leu Glu Asp
   610 615 620
   Pro Glu Phe Gln Ala Ser Asn Ile Met His Ser Ile Asn Gly Tyr Val
   625 630 635 640
   Phe Asp Ser Leu Gln Leu Ser Val Cys Leu His Glu Val Ala Tyr Trp
   645 650 655
   Tyr Ile Leu Ser Ile Gly Ala Gln Thr Asp Phe Leu Ser Val Phe Phe
   660 665 670
   Ser Gly Tyr Thr Phe Lys His Lys Met Val Tyr Glu Asp Thr Leu Thr
   675 680 685
   Leu Phe Pro Phe Ser Gly Glu Thr Val Phe Met Ser Met Glu Asn Pro
   690 695 700
   Gly Leu Trp Ile Leu Gly Cys His Asn Ser Asp Phe Arg Asn Arg Gly
   705                 710                 715                 720
   Met Thr Ala Leu Leu Lys Val Ser Ser Cys Asp Lys Asn Thr Gly Asp
                   725                 730                 735
   Tyr Tyr Glu Asp Ser Tyr Glu Asp Ile Ser Ala Tyr Leu Leu Ser Lys
               740                 745                 750
   Asn Asn Ala Ile Glu Pro Arg Ser Phe Ser Gln Gln Asn Pro Pro Val
           755                 760                 765
   Leu Lys Arg His Gln Arg Glu Ile Thr Arg Thr Thr Leu Gln Ser Asp
       770                 775                 780
   Gln Glu Glu Ile Asp Tyr Asp Asp Thr Ile Ser Val Glu Met Lys Lys
   785                 790                 795                 800
   Glu Asp Phe Asp Ile Tyr Asp Glu Asp Glu Asn Gln Ser Pro Arg Ser
                   805                 810                 815
   Phe Gln Lys Lys Thr Arg His Tyr Phe Ile Ala Ala Val Glu Arg Leu
               820                 825                 830
   Trp Asp Tyr Gly Met Ser Ser Ser Pro His Val Leu Arg Asn Arg Ala
           835                 840                 845
   Gln Ser Gly Ser Val Pro Gln Phe Lys Lys Val Val Phe Gln Glu Phe
       850                 855                 860
   Thr Asp Gly Ser Phe Thr Gln Pro Leu Tyr Arg Gly Glu Leu Asn Glu
   865                 870                 875                 880
   His Leu Gly Leu Leu Gly Pro Tyr Ile Arg Ala Glu Val Glu Asp Asn
                   885                 890                 895
   Ile Met Val Thr Glu Asp Gln Arg Gln Gly Ala Glu Pro Arg Lys Asn
               900                 905                 910
   Phe Val Lys Pro Asn Glu Thr Lys Thr Tyr Phe Trp Lys Val Gln His
           915                 920                 925
   His Met Ala Pro Thr Lys Asp Glu Phe Asp Cys Lys Ala Trp Ala Tyr
       930                 935                 940
   Phe Ser Asp Val Asp Leu Glu Lys Asp Val His Ser Gly Leu Ile Gly
   945                 950                 955                 960
   Pro Leu Leu Val Cys His Thr Asn Thr Leu Asn Pro Ala His Gly Arg
   965 970 975
   Gln Val Thr Val Gln Glu Phe Ala Leu Phe Phe Thr Ile Phe Asp Glu
   980 985 990
   Thr Lys Ser Trp Tyr Phe Thr Glu Asn Met Glu Arg Asn Cys Arg Ala
   995 1000 1005
   Pro Cys Asn Ile Gln Met Glu Asp Pro Thr Phe Lys Glu Asn Tyr
   1010 1015 1020
   Arg Phe His Ala Ile Asn Gly Tyr Ile Met Asp Thr Leu Pro Gly
   1025 1030 1035
   Leu Val Met Ala Gln Asp Gln Arg Ile Arg Trp Tyr Leu Leu Ser
   1040 1045 1050
   Met Gly Ser Asn Glu Asn Ile His Ser Ile His Phe Ser Gly His
   1055 1060 1065
   Val Phe Thr Val Arg Lys Lys Glu Glu Tyr Lys Met Ala Leu Tyr
   1070 1075 1080
   Asn Leu Tyr Pro Gly Val Phe Glu Thr Val Glu Met Leu Pro Ser
   1085 1090 1095
   Lys Ala Gly Ile Trp Arg Val Glu Cys Leu Ile Gly Glu His Leu
   1100 1105 1110
   His Ala Gly Met Ser Thr Leu Phe Leu Val Tyr Ser Asn Lys Cys
   1115 1120 1125
   Gln Thr Pro Leu Gly Met Ala Ser Gly His Ile Arg Asp Phe Gln
   1130 1135 1140
   Ile Thr Ala Ser Gly Gln Tyr Gly Gln Trp Ala Pro Lys Leu Ala
   1145 1150 1155
   Arg Leu His Tyr Ser Gly Ser Ile Asn Ala Trp Ser Thr Lys Glu
   1160 1165 1170
   Pro Phe Ser Trp Ile Lys Val Asp Leu Leu Ala Pro Met Ile Ile
   1175 1180 1185
   His Gly Ile Lys Thr Gln Gly Ala Arg Gln Lys Phe Ser Ser Leu
   1190 1195 1200
   Tyr Ile  Ser Gln Phe Ile Ile  Met Tyr Ser Leu Asp  Gly Lys Lys
       1205                 1210                 1215
   Trp Gln  Thr Tyr Arg Gly Asn  Ser Thr Gly Thr Leu  Met Val Phe
       1220                 1225                 1230
   Phe Gly  Asn Val Asp Ser Ser  Gly Ile Lys His Asn  Ile Phe Asn
       1235                 1240                 1245
   Pro Pro  Ile Ile Ala Arg Tyr  Ile Arg Leu His Pro  Thr His Tyr
       1250                 1255                 1260
   Ser Ile  Arg Ser Thr Leu Arg  Met Glu Leu Met Gly  Cys Asp Leu
       1265                 1270                 1275
   Asn Ser  Cys Ser Met Pro Leu  Gly Met Glu Ser Lys  Ala Ile Ser
       1280                 1285                 1290
   Asp Ala  Gln Ile Thr Ala Ser  Ser Tyr Phe Thr Asn  Met Phe Ala
       1295                 1300                 1305
   Thr Trp  Ser Pro Ser Lys Ala  Arg Leu His Leu Gln  Gly Arg Ser
       1310                 1315                 1320
   Asn Ala  Trp Arg Pro Gln Val  Asn Asn Pro Lys Glu  Trp Leu Gln
       1325                 1330                 1335
   Val Asp  Phe Gln Lys Thr Met  Lys Val Thr Gly Val  Thr Thr Gln
       1340                 1345                 1350
   Gly Val  Lys Ser Leu Leu Thr  Ser Met Tyr Val Lys  Glu Phe Leu
       1355                 1360                 1365
   Ile Ser  Ser Ser Gln Asp Gly  His Gln Trp Thr Leu  Phe Phe Gln
       1370                 1375                 1380
   Asn Gly  Lys Val Lys Val Phe  Gln Gly Asn Gln Asp  Ser Phe Thr
       1385                 1390                 1395
   Pro Val  Val Asn Ser Leu Asp  Pro Pro Leu Leu Thr  Arg Tyr Leu
       1400                 1405                 1410
   Arg Ile  His Pro Gln Ser Trp  Val His Gln Ile Ala  Leu Arg Met
       1415                 1420                 1425
   Glu Val  Leu Gly Cys Glu Ala  Gln Asp Leu Tyr
       1430                 1435